# EUROPEAN PATENT SPECIFICATION

(11) **EP 0 524 958 B1**
(45) Date of publication and mention of the grant of the patent: **18.09.1996**
(21) Application number: 91906028.5
(22) Date of filing: 05.03.1991
(51) Int. Cl.: A61K 39/00, C12Q 1/00, A01N 37/18, C07K 14/20, C07K 16/12

(54) **ANTIGENIC PROTEINS OF BORRELIA BURGDORFERI**
ANTIGEN-WIRKENDE PROTEINE VON BORRELIA BURGDORFERI
PROTEINES ANTIGENIQUES DE BORRELIA BURGDORFERI

(30) Priority: 05.03.1990 US 487716
(43) Date of publication of application: 03.02.1993
(73) Proprietor: THE UNITED STATES OF AMERICA as represented by the Secretary UNITED STATES DEPARTMENT OF COMMERCE, Washington, DC 20231 (US)
(72) Inventor: SIMPSON, Warren J., North East Valley, Dunedin (NZ); SCHWAN, Tom, G., Hamilton, MT 59840 (US)
(74) Representative: Perry, Robert Edward
(86) International application number: US9101500
(87) International publication number: WO9113630

(56) References cited:
- JOURNAL OF CLINICAL MICROBIOLOGY, vol. 28, no. 6, June 1990; W.J. SIMPSON et al., pp. 1329-1337/
- JOURNAL OF CLINICAL MICROBIOLOGY, vol. 29, no. 2, February 1991; W.J. SIMPSON et al., pp. 236-243/
- INFECTION & IMMUNITY, vol. 54, no. 1, October 1986; T.R. HOWE et al., pp. 207- 212/
- JOURNAL OF CLINICAL MICROBIOLOGY, vol. 27, no. 8, August 1989; K.E. HECHEMY et
- al., pp. 1854-1858/
- REVIEW OF INFECTIOUS DISEASES, vol. 11, supplement 6, September-October 1989; A.G. BARBOUR, pp. S1470-S1474/
- Serodiagnosis Immunotherapy Infectious Diseases 2 (1988) 375
- Pediatric Research 26 (1989) 377

SEQUENCE LISTING
(1) GENERAL INFORMATION:
(i) APPLICANT:
(ii) TITLE OF INVENTION: ANTIGENIC PROTEINS OF
BORRELIA BURGDORFERI
(iii) NUMBER OF SEQUENCES: 7
(iv) CORRESPONDENCE ADDRESS:
(A) ADDRESSEE:
(B) STREET:
(C) CITY:
(D) STATE:
(E) COUNTRY:
(F) ZIP:
(v) COMPUTER READABLE FORM:
(A) MEDIUM TYPE: Diskette - 3.5 inches,
1.44 Mb storage
(B) COMPUTER: IBM PC Compatible
(C) OPERATING SYSTEM: MS-DOS
(D) SOFTWARE: WordPerfect 5.0
(vi) CURRENT APPLICATION DATA:
(A) APPLICATION NUMBER:
(B) FILING DATE:
(C) CLASSIFICATION:
(vii) PRIOR APPLICATION DATA:
(A) APPLICATION NUMBER:
(B) FILING DATE:
(viii) ATTORNEY/AGENT INFORMATION:
(A) NAME:
(B) REGISTRATION NUMBER:
(C) REFERENCE/DOCKET NUMBER:
(ix) TELECOMMUNICATION INFORMATION:
(A) TELEPHONE:
(B) TELEFAX:
(C) TELEX:

(2) INFORMATION FOR SEQ ID NO: 1:
(i) SEQUENCE CHARACTERISTICS:
(A) LENGTH: 2,307
(B) TYPE: Nucleic Acid
(C) STRANDEDNESS: double stranded
(D) TOPOLOGY: Linear
(ii) MOLECULE TYPE: Genomic DNA
(A) DESCRIPTION:
(iii) HYPOTHETICAL:
(iv) ANTI-SENSE:
(v) FRAGMENT TYPE:
(vi) ORIGINAL SOURCE:
(A) ORGANISM: Borrelia burgdorferi
(B) STRAIN: Sh-2-82
(C) INDIVIDUAL ISOLATE: Sh-2-82, P-6
(D) DEVELOPMENTAL STAGE:
(E) HAPLOTYPE:
(F) TISSUE TYPE:
(G) CELL TYPE:
(H) CELL LINE:
(I) ORGANELLE:
(vii) IMMEDIATE SOURCE:
(A) LIBRARY:
(B) CLONE: pSPR33
(viii) POSITION IN GENOME:
(A) CHROMOSOME/SEGMENT:
(B) MAP POSITION:
(C) UNITS:
(ix) FEATURE:
(A) NAME/KEY:
(B) LOCATION:
(C) IDENTIFICATION METHOD: By experiment
(D) OTHER INFORMATION: Expression of P-39
antigens
(x) PUBLICATION INFORMATION:
(A) AUTHORS:
(B) TITLE:
(C) JOURNAL:

(D) VOLUME:
(E) ISSUE:
(F) PAGES:
(G) DATE:
(H) DOCUMENT NUMBER:
(I) FILING DATE:
(J) PUBLICATION DATE:
(K) RELEVANT RESIDUES:
(xi) SEQUENCE DESCRIPTION: SEQ ID NO: 1:
TCCTGATAGT GAATATGCAT TTGATTTATT TAAATCAAAG TTATAAACTA CTAAATATAG 60
-35 -10
CTTTGTTTGT AAAGGGGAAA TAGTTT ATG AAT AAA ATA TTG TTG TTG ATT TTG 113
Met Asn Lys Ile Leu Leu Leu Ile Leu
1
CTT GAG AGT ATT GTT TTT TTA TCT TGT AGT GGT AAA GGT AGT CTT GGG 161
Leu Glu Ser Ile Val Phe Leu Ser Cys Ser Gly Lys Gly Ser Leu Gly
10 20
AGC GAA ATT CCT AAG GTA TCT TTA ATA ATT GAT GGA ACT TTT GAT GAT 209
Ser Glu Ile Pro Lys Val Ser Leu Ile Ile Asp Gly Thr Phe Asp Asp
30 40
AAA TCT TTT AAT GAG AGT GCT TTA AAT GGC GTA AAA AAA GTT AAA GAA 257
Lys Ser Phe Asn Glu Ser Ala Leu Asn Gly Val Lys Lys Val Lys Glu
50
GAA TTT AAA ATT GAG CTT GTT TTA AAA GAA TCC TCA TCA AAT TCT TAT 305
Glu Phe Lys Ile Glu Leu Val Leu Lys Glu Ser Ser Ser Asn Ser Tyr
60 70
TTA TCT GAT CTT GAA GGG CTT AAG GAT GCG GGC TCA GAT TTA ATT TGG 353
Leu Ser Asp Leu Glu Gly Leu Lys Asp Ala Gly Ser Asp Leu Ile Trp
80
CTT ATT GGG TAT AGA TTT AGC GAT GTG GCC AAG GTT GCG GCT CTT 401
Leu Ile Gly Tyr Agr Phe Ser Asp Val Ala Lys Val Ala Ala Leu
90 100
CAA AAT CCC GAT ATG AAA TAT GCA ATT ATT GAT CCT ATT TAT TCT AAC 449
Gln Asn Pro Asp Met Lys Tyr Ala Ile Ile Asp Pro Ile Tyr Ser Asn
110 120
GAT CCT ATT CCT GCA AAT TTG GTG GGC ATG ACC TTT AGA GCT GAA GAG 497
Asp Pro Ile Pro Ala Asn Leu Val Gly Met Thr Phe Arg Ala Gln Glu
130
GGT GCA TTT TTA ACG GGT TAT ATT GCT GCA AAA CTT TCT AAA ACA GGT 545
Gly Ala Phe Leu Thr Gly Tyr Ile Ala Ala Lys Leu Ser Lys Thr Gly
140 150

AAA ATT GGA TTT TTA GGG GGA ATA GAA GGC GAG ATA GTA GAT GCT TTT 593
Lys Ile Gly Phe Leu Gly Gly Ile Glu Gly Glu Ile Val Asp Ala Phe
160

AGG TAT GGG TAT GAA GCT GGT GCT AAG TAT GCT AAT AAA GAT ATA AAG 641
Arg Tyr Gly Tyr Glu Ala Gly Ala Lys Try Ala Asn Lys Asp Ile Lys
170 180

ATA TCT ACT CAG TAT ATT GGT AGT TTT GCT GAC CTT GAA GCT GGT AGA 689
Ile Ser Thr Gln Tyr Ile Gly Ser Phe Ala Asp Leu Glu Ala Gly Arg
190 200

AGC GTT GCA ACT AGA ATG TAT TCT GAT GAG ATA GAC ATT ATT CAT CAT 737
Ser Val Ala Thr Arg Met Try Ser Asp Glu Ile Asp Ile Ile His His
210

GCT GCA GGC CTT GGA GGA ATT GGG GCT ATT GAG GTT GCA AAA GAA CTT 785
Ala Ala Gly Leu Gly Gly Ile Gly Ala Ile Glu Val Ala Lys Glu Leu
220 230

GGT TCT GGG CAT TAC ATT ATT GGA GTT GAT GAA GAT CAA GCA TAT CTT 833
Gly Ser Gly His Tyr Ile Ile Gly Val Asp Glu Asp Gln Ala Tyr Leu
240

GCT CCT GAC AAT GTA ATA ACA TCT ACA ACT AAA GAT GTT GGT AGA GCT 881
Ala Pro Asp Asn Val Ile Thr Ser Thr Thr Lys Asp Val Gly Arg Ala
250 260

TTA AAT ATT TTT ACA TCT AAC CAT TTA AAA ACT AAT ACT TTC GAA GGT 929
Leu Asn Ile Phe Thr Ser Asn His Leu Lys Thr Asn Thr Phe Glu Gly
270 .280

GGC AAA TTA ATA AAT TAT GGC CTT AAA GAA GGA GTT GTG GGG TTT GTA 977
Gly Lys Leu Ile Asn Tyr Gly Leu Lys Glu Gly Val Val Gly Phe Val
290

AGA AAT CCT AAA ATG ATT TCC TTT GAA CTT GAA AAA GAA ATT GAC AAT 1025
Arg Asn Pro Lys Met Ile Ser Phe Glu Leu Glu Lys Glu Ile Asp Asn
300 310

CTT TCT AGC AAA ATA ATC AAC AAA GAA ATT ATT GTT CCA TCT AAT AAA 1073
Leu Ser Ser Lys Ile Ile Asn Lys Glu Ile Ile Val Pro Ser Asn Lys
320

GAA AGT TAT GAG AAG TTT CTT AAA GAA TTT ATT TAA ATAAAGAATC AATTTATATA
1129 Gly Ser Tyr Glu Lys Phe Leu Lys Glu Phe Ile ***
330

TTTTATTTTT AAGTATAAAA AACACATTGG TTTTGTTTGA ATAATTGAAA TGGAGAAGTG 1189

CTTTAT ATG AGA ATT GTA ATT TTT ATA TTC GGT ATT TTT TTG ACT TCT 1237
Met Arg Ile Val Ile Phe Ile Phe Gly Ile Leu Leu Thr Ser
1 10

TGC TTT AGT AGA AAT GGA ATA GAA TCT AGT TCA AAA AAA ATT AAG ATA 1285
Cys Phe Ser Arg Asn Gly Ile Gly Ser Ser Ser Lys Lys Ile Lys Ile
20 30

TCC ATG TTG GTA GAT GGT GTT CTT GAC GAC AAA TCT TTT AAT TCT AGT 1333
Ser Met Leu Val Asp Gly Val Leu Asp Asp Lys Ser Phe Asn Ser Ser
40

GCT AAT GAG GCT TTA TTA CGC TTG AAA AAA GAT TTT CCA GAA AAT ATT 1381
Ala Asn Glu Ala Leu Leu Arg Leu Lys Lys Asp Phe Pro Glu Asn Ile
50 60

GAA GAA GTT TTT TCT TGT GCT ATT TCT GGA GTT TAT TCT AGT TAT GTT 1429
Glu Glu Val Phe Ser Cys Ala Ile Ser Gly Val Tyr Ser Ser Tyr Val
70

TCA GAT CTT GAT AAT TTA AAA AGG AAT GGC TCA GAC TTG ATT TGG CTT 1477
Ser Asp Leu Asp Asn Leu Lys Arg Asn Gly Ser Asp Leu Ile Trp Leu
80 90

GTA GGG TAC ATG CTT ACG GAT GCA TCT TTA TTG GTT TCA TCG GAG AAT 1525
Val Gly Tyr Met Leu Thr Asp Ala Ser Leu Leu Val Ser Ser Glu Asn
100 110

CCA AAA ATT AGC TAT GGA ATA ATA GAT CCC ATT TAT GGT GAT GAT GTT 1573
Pro Lys Ile Ser Tyr Gly Ile Ile Asp Pro Ile Tyr Gly Asp Asp Val
120

CAG ATT CCT GAA AAC TTG ATT GCT GTT GTT TTC AGA GTA GAG CAA GGT 1621
Gln Ile Pro Glu Asn Leu Ile Ala Val Val Phe Arg Val Glu Gln Gly
130 140

GCT TTT TTG GCT GGC TAT ATT GAC GCC AAA AAA AGC TTT TCT GGC AAA 1669
Ala Phe Leu Ala Gly Thr Ile Ala Ala Lys Lys Ser Phe Ser Gly Lys
150

ATA GGT TTT ATA GGG GGA ATG AAG GGT AAT ATA GTA GAT GCA TTT CGC 1717
Ile Gly Phe Ile Gly Gly Met Lys Gly Asn Ile Val Asp A[?][?] Phe Ar[?]
170

[?][?][?] [?][?][?] [?]A[?] GAA TCT GGA GCA AAG TAT GCT AAT AAA GAT ATA GAG ATT 1765
Thr Gly Tyr Glu Ser Gly Ala Lys Tyr Ala Asn Lys Asp Ile Glu Ile
180 190

ATA AGT GAA TAT TCC AAT TCT TTT TCC GAT GTT GAT ATT GGT AGA ACC 1813
Ile Ser Glu Tyr Ser Asn Ser Phe Ser Asp Val Asp Ile Gly Arg Thr
200

ATA GCT AGT AAA ATG TAT TCT AAA GGG ATA GAT GTA ATT CAT TTT GCA 1861
Ile Ala Ser Lys Met Tyr Ser Lys Gly Ile Asp Val Ile His Phe Ala
210 220

GCT GGT TTA GCA GGA ATT GGT GTT ATT GAG GCA GCA AAA AAC CTT GGC 1909
Ala Gly Leu Ala Gly Ile Gly Val Ile Glu Ala Ala Lys Asn Leu Gly
230

GAT GGT TAC TAT GTT ATT GGA GCC GAT CAG GAT CAG TCA TAT CTT GCT 1957
Asp Gly Tyr Tyr Val Ile Gly Ala Asp Gln Asp Gln Ser Tyr Leu Ala
240 250

CCT AAA AAT TTT ATT ACT TCT GTT ATA AAA AAC ATT GGG GAC GCA TTG 2005
Pro Lys Asn Phe Ile Thr Ser Val Ile Lys Asn Ile Gly Asp Ala Leu
260 270

TAT TTG ATT ACT GGC GAA TAT ATT AAA AAT AAT AAT GTT TGG GAA GGT 2053
Tyr Leu Ile Thr Gly Glu Tyr Ile Lys Asn Asn Asn Val Trp Glu Gly
280

GGA AAA GTT GTT CAA ATG GGA TTA AGA GAT GGT GTT ATT GGG CTG CCT 2101
Gly Lys Val Val Gln Met Gly Leu Arg Asp Gly Val Ile Gly Leu Pro
290 300

AAT GCG AAT GAA TTT GAA TAC ATA AAA GTT CTT GAG AGA AAA ATA GTC 2149
Asn Ala Asn Glu Phe Glu Tyr Ile Lys Val Leu Glu Arg Lys Ile Val
310

AAT AAA GAG ATC ATT GTT CCT TGC AAT CAG GAG GAA TAT GAA ATT TTT 2197
Asn Lys Glu Ile Ile Val Pro Cys Asn Gln Glu Glu Tyr Glu Asn Phe
320 330

ATA AAA CAA ATA TTA AAG TTA TAA ACTTTTGAAA TAGAAAGATT TTAATTTTCC 2251
Ile Lys Gln Ile Leu Lys Leu ***
340

AGTTTTTAAT TTTTTAATTA TGTTATATTT ATTGTGTTAT AATAAATAGA AGTACA 2307

(2) INFORMATION FOR SEQ ID NO: 2:
(i) SEQUENCE CHARACTERISTICS:
(A) LENGTH: 1109
(B) TYPE: Nucleic Acid
(C) STRANDEDNESS: double stranded
(D) TOPOLOGY: Linear
(ii) MOLECULE TYPE: Genomic DNA
(A) DESCRIPTION:
(iii) HYPOTHETICAL:
(iv) ANTI-SENSE:
(v) FRAGMENT TYPE:
(vi) ORIGINAL SOURCE:
(A) ORGANISM: Borrelia burgdorferi
(B) STRAIN: Sh-2-82
(C) INDIVIDUAL ISOLATE: Sh-2-82, P-6
(D) DEVELOPMENTAL STAGE:
(E) HAPLOTYPE:

(F) TISSUE TYPE:
(G) CELL TYPE:
(H) CELL LINE:
(I) ORGANELLE:
(vii) IMMEDIATE SOURCE:
(A) LIBRARY:
(B) CLONE: pSPR33
(viii) POSITION IN GENOME:
(A) CHROMOSOME/SEGMENT:
(B) MAP POSITION:
(C) UNITS:
(ix) FEATURE:
(A) NAME/KEY:
(B) LOCATION:
(C) IDENTIFICATION METHOD: By experiment
(D) OTHER INFORMATION:
(x) PUBLICATION INFORMATION:
(A) AUTHORS:
(B) TITLE:
(C) JOURNAL:
(D) VOLUME:
(E) ISSUE:
(F) PAGES:
(G) DATE:
(H) DOCUMENT NUMBER:
(I) FILING DATE:
(J) PUBLICATION DATE:
(K) RELEVANT RESIDUES:
(xi) SEQUENCE DESCRIPTION: SEQ ID NO: 2:
TCCTGATAGT GAATATGCAT TTGATTTATT TAAATCAAAG TTATAAACTA CTAAATATAG 60
-35 -10

CTTTGTTTGT AAAGGGGAAA TAGTTT ATG AAT AAA ATA TTG TTG TTG ATT TTG 113
Met Asn Lys Ile Leu Leu Leu Ile Leu
1

CTT GAG AGT ATT GTT TTT TTA TCT TGT AGT GGT AAA GGT AGT CTT GGG 161
Leu Glu Ser Ile Val Phe Leu Ser Cys Ser Gly Lys Gly Ser Leu Gly
10 20

AGC GAA ATT CCT AAG GTA TCT TTA ATA ATT GAT GGA ACT TTT GAT GAT 209
Ser Glu Ile Pro Lys Val Ser Leu Ile Ile Asp Gly Thr Phe Asp Asp
30 40

AAA TCT TTT AAT GAG AGT GCT TTA AAT GGC GTA AAA AAA GTT AAA GAA 257
Lys Ser Phe Asn Glu Ser Ala Leu Asn Gly Val Lys Lys Val Lys Glu
50

GAA TTT AAA ATT GAG CTT GTT TTA AAA GAA TCC TCA TCA AAT TCT TAT 305
Glu Phe Lys Ile Glu Leu Val Leu Lys Glu Ser Ser Ser Asn Ser Tyr
60 70

TTA TCT GAT CTT GAA GGG CTT AAG GAT GCG GGC TCA GAT TTA ATT TGG 353
Leu Ser Asp Leu Glu Gly Leu Lys Asp Ala Gly Ser Asp Leu Ile Trp
80

CTT ATT GGG TAT AGA TTT AGC GAT GTG GCC AAG GTT GCG GCT CTT 401
Leu Ile Gly Tyr Agr Phe Ser Asp Val Ala Lys Val Ala Ala Leu
90 100

CAA AAT CCC GAT ATG AAA TAT GCA ATT ATT GAT CCT ATT TAT TCT AAC 449
Gln Asn Pro Asp Met Lys Tyr Ala Ile Ile Asp Pro Ile Tyr Ser Asn
110 120

GAT CCT ATT CCT GCA AAT TTG GTG GGC ATG ACC TTT AGA GCT GAA GAG 497
Asp Pro Ile Pro Ala Asn Leu Val Gly Met Thr Phe Arg Ala Gln Glu
130

GGT GCA TTT TTA ACG GGT TAT ATT GCT GCA AAA CTT TCT AAA ACA GGT 545
Gly Ala Phe Leu Thr Gly Tyr Ile Ala Ala Lys Leu Ser Lys Thr Gly
140 150

AAA ATT GGA TTT TTA GGG GGA ATA GAA GGC GAG ATA GTA GAT GCT TTT 593
Lys Ile Gly Phe Leu Gly Gly Ile Glu Gly Glu Ile Val Asp Ala Phe
160

AGG TAT GGG TAT GAA GCT GGT GCT AAG TAT GCT AAT AAA GAT ATA AAG 641
Arg Tyr Gly Tyr Glu Ala Gly Ala Lys Try Ala Asn Lys Asp Ile Lys
170 180

ATA TCT ACT CAG TAT ATT GGT AGT TTT GCT GAC CTT GAA GCT GGT AGA 689
Ile Ser Thr Gln Tyr Ile Gly Ser Phe Ala Asp Leu Glu Ala Gly Arg
190 200

AGC GTT GCA ACT AGA ATG TAT TCT GAT GAG ATA GAC ATT ATT CAT CAT 737
Ser Val Ala Thr Arg Met Try Ser Asp Glu Ile Asp Ile Ile His His
210

GCT GCA GGC CTT GGA GGA ATT GGG GCT ATT GAG GTT GCA AAA GAA CTT 785
Ala Ala Gly Leu Gly Gly Ile Gly Ala Ile Glu Val Ala Lys Glu Leu
220 230

GGT TCT GGG CAT TAC ATT ATT GGA GTT GAT GAA GAT CAA GCA TAT CTT 833
Gly Ser Gly His Tyr Ile Ile Gly Val Asp Glu Asp Gln Ala Tyr Leu
240

GCT CCT GAC AAT GTA ATA ACA TCT ACA ACT AAA GAT GTT GGT AGA GCT 881
Ala Pro Asp Asn Val Ile Thr Ser Thr Thr Lys Asp Val Gly Arg Ala
250 260

TTA AAT ATT TTT ACA TCT AAC CAT TTA AAA ACT AAT ACT TTC GAA GGT 929
Leu Asn Ile Phe Thr Ser Asn His Leu Lys Thr Asn Thr Phe Glu Gly
270 280

GGC AAA TTA ATA AAT TAT GGC CTT AAA GAA GGA GTT GTG GGG TTT GTA 977
Gly Lys Leu Ile Asn Tyr Gly Leu Lys Glu Gly Val Val Gly Phe Val
290

AGA AAT CCT AAA ATG ATT TCC TTT GAA CTT GAA AAA GAA ATT GAC AAT 1025
Arg Asn Pro Lys Met Ile Ser Phe Glu Leu Glu Lys Glu Ile Asp Asn
300 310

CTT TCT AGC AAA ATA ATC AAC AAA GAA ATT ATT GTT CCA TCT AAT AAA 1073
Leu Ser Ser Lys Ile Ile Asn Lys Glu Ile Ile Val Pro Ser Asn Lys
320

GAA AGT TAT GAG AAG TTT CTT AAA GAA TTT ATT TAA 1109
Gly Ser Tyr Glu Lys Phe Leu Lys Glu Phe Ile ***

(2) INFORMATION FOR SEQ ID NO: 3:
(i) SEQUENCE CHARACTERISTICS:
(A) LENGTH: 1198
(B) TYPE: Nucleic Acid
(C) STRANDEDNESS: double stranded
(D) TOPOLOGY: Linear
(ii) MOLECULE TYPE: Genomic DNA
(A) DESCRIPTION:
(iii) HYPOTHETICAL:
(iv) ANTI-SENSE:
(v) FRAGMENT TYPE:
(vi) ORIGINAL SOURCE:
(A) ORGANISM: Borrelia burgdorferi
(B) STRAIN: Sh-2-82
(C) INDIVIDUAL ISOLATE: Sh-2-82, P-6
(D) DEVELOPMENTAL STAGE:
(E) HAPLOTYPE:
(F) TISSUE TYPE:
(G) CELL TYPE:
(H) CELL LINE:
(I) ORGANELLE:

(vii) IMMEDIATE SOURCE:
(A) LIBRARY:
(B) CLONE: pSPR33
(viii) POSITION IN GENOME:
(A) CHROMOSOME/SEGMENT:
(B) MAP POSITION:
(C) UNITS:
(ix) FEATURE:
(A) NAME/KEY:
(B) LOCATION:
(C) IDENTIFICATION METHOD:
(D) OTHER INFORMATION:
(x) PUBLICATION INFORMATION:
(A) AUTHORS:
(B) TITLE:
(C) JOURNAL:
(D) VOLUME:
(E) ISSUE:
(F) PAGES:
(G) DATE:
(H) DOCUMENT NUMBER:
(I) FILING DATE:
(J) PUBLICATION DATE:
(K) RELEVANT RESIDUES:
(xi) SEQUENCE DESCRIPTION: SEQ ID NO: 3:
ATAAAGAATC AATTTATATA 20
TTTTATTTTT AAGTATAAAA AACACATTGG TTTTGTTTGA ATAATTGAAA TGGAGAAGTG 80
CTTTAT ATG AGA ATT GTA ATT TTT ATA TTC GGT ATT TTT TTG ACT TCT 128
Met Arg Ile Val Ile Phe Ile Phe Gly Ile Leu Leu Thr Ser
1 10
TGC TTT AGT AGA AAT GGA ATA GAA TCT AGT TCA AAA AAA ATT AAG ATA 176
Cys Phe Ser Arg Asn Gly Ile Gly Ser Ser Ser Lys Lys Ile Lys Ile
20 30
TCC ATG TTG GTA GAT GGT GTT CTT GAC GAC AAA TCT TTT AAT TCT AGT 224
Ser Met Leu Val Asp Gly Val Leu Asp Asp Lys Ser Phe Asn Ser Ser
40
GCT AAT GAG GCT TTA TTA CGC TTG AAA AAA GAT TTT CCA GAA AAT ATT 272
Ala Asn Glu Ala Leu Leu Arg Leu Lys Lys Asp Phe Pro Glu Asn Ile
50 60

GAA GAA GTT TTT TCT TGT GCT ATT TCT GGA GTT TAT TCT AGT TAT GTT 320
Glu Glu Val Phe Ser Cys Ala Ile Ser Gly Val Tyr Ser Ser Tyr Val
70

TCA GAT CTT GAT AAT TTA AAA AGG AAT GGC TCA GAC TTG ATT TGG CTT 368
Ser Asp Leu Asp Asn Leu Lys Arg Asn Gly Ser Asp Leu Ile Trp Leu
80 90

GTA GGG TAC ATG CTT ACG GAT GCA TCT TTA TTG GTT TCA TCG GAG AAT 416
Val Gly Tyr Met Leu Thr Asp Ala Ser Leu Leu Val Ser Ser Glu Asn
100 110

CCA AAA ATT AGC TAT GGA ATA ATA GAT CCC ATT TAT GGT GAT GAT GTT 464
Pro Lys Ile Ser Tyr Gly Ile Ile Asp Pro Ile Tyr Gly Asp Asp Val
120

CAG ATT CCT GAA AAC TTG ATT GCT GTT GTT TTC AGA GTA GAG CAA GGT 512
Gln Ile Pro Glu Asn Leu Ile Ala Val Val Phe Arg Val Glu Gln Gly
130 140

GCT TTT TTG GCT GGC TAT ATT GAC GCC AAA AAA AGC TTT TCT GGC AAA 560
Ala Phe Leu Ala Gly Thr Ile Ala Ala Lys Lys Ser Phe Ser Gly Lys
150

ATA GGT TTT ATA GGG GGA ATG AAG GGT AAT ATA GTA GAT GCA TTT CGC 608
Ile Gly Phe Ile Gly Gly Met Lys Gly Asn Ile Val Asp Ala Phe Arg
160 170

TAT GGT TAT GAA TCT GGA GCA AAG TAT GCT AAT AAA GAT ATA GAG ATT 656
Thr Gly Tyr Glu Ser Gly Ala Lys Tyr Ala Asn Lys Asp Ile Glu Ile
180 190

ATA AGT GAA TAT TCC AAT TCT TTT TCC GAT GTT GAT ATT GGT AGA ACC 704
Ile Ser Glu Tyr Ser Asn Ser Phe Ser Asp Val Asp Ile Gly Arg Thr
200

ATA GCT AGT AAA ATG TAT TCT AAA GGG ATA GAT GTA ATT CAT TTT GCA 752
Ile Ala Ser Lys Met Tyr Ser Lys Gly Ile Asp Val Ile His Phe Ala
210 220

GCT GGT TTA GCA GGA ATT GGT GTT ATT GAG GCA GCA AAA AAC CTT GGC 800
Ala Gly Leu Ala Gly Ile Gly Val Ile Glu Ala Ala Lys Asn Leu Gly
230

GAT GGT TAC TAT GTT ATT GGA GCC GAT CAG GAT CAG TCA TAT CTT GCT 848
Asp Gly Tyr Tyr Val Ile Gly Ala Asp Gln Asp Gln Ser Tyr Leu Ala
240 250

CCT AAA AAT TTT ATT ACT TCT GTT ATA AAA AAC ATT GGG GAC GCA TTG 896
Pro Lys Asn Phe Ile Thr Ser Val Ile Lys Asn Ile Gly Asp Ala Leu
260 270

TAT TTG ATT ACT GGC GAA TAT ATT AAA AAT AAT AAT GTT TGG GAA GGT 944
Tyr Leu Ile Thr Gly Glu Tyr Ile Lys Asn Asn Asn Val Trp Glu Gly
280

GGA AAA GTT GTT CAA ATG GGA TTA AGA GAT GGT GTT ATT GGG CTG CCT 992
Gly Lys Val Val Gln Met Gly Leu Arg Asp Gly Val Ile Gly Leu Pro
290 300

AAT GCG AAT GAA TTT GAA TAC ATA AAA GTT CTT GAG AGA AAA ATA GTC 1040
Asn Ala Asn Glu Phe Glu Tyr Ile Lys Val Leu Glu Arg Lys Ile Val
310

AAT AAA GAG ATC ATT GTT CCT TGC AAT CAG GAG GAA TAT GAA ATT TTT 1088
Asn Lys Glu Ile Ile Val Pro Cys Asn Gln Glu Glu Tyr Glu Asn Phe
320 330

ATA AAA CAA ATA TTA AAG TTA TAA ACTTTTGAAA TAGAAAGATT TTAATTTTCC 1142
Ile Lys Gln Ile Leu Lys Leu ***
340

AGTTTTTAAT TTTTTAATTA TGTTATATTT ATTGTGTTAT AATAAATAGA AGTACA 1198
(2) INFORMATION FOR SEQ ID NO: 4:
(i) SEQUENCE CHARACTERISTICS:
(A) LENGTH: 18
(B) TYPE: Nucleic Acid
(C) STRANDEDNESS: Single-Stranded
(D) TOPOLOGY: Linear
(ii) MOLECULE TYPE: Synthetic DNA
(A) DESCRIPTION: A primer from the flanking
sequence of 5' to gene 1 of P-39 in B. burgdorferi.
(iii) HYPOTHETICAL:
(iv) ANTI-SENSE:
(v) FRAGMENT TYPE:
(vi) ORIGINAL SOURCE:
(A) ORGANISM: B. burgdorferi
(B) STRAIN: Sh-2-82
(C) INDIVIDUAL ISOLATE: Sh-2-82, P-6
(D) DEVELOPMENTAL STAGE:
(E) HAPLOTYPE:
(F) TISSUE TYPE:
(G) CELL TYPE:
(H) CELL LINE:
(I) ORGANELLE:
(vii) IMMEDIATE SOURCE:
(A) LIBRARY:
(B) CLONE: pSPR33

(viii) POSITION IN GENOME:
(A) CHROMOSOME/SEGMENT:
(B) MAP POSITION:
(C) UNITS:
(ix) FEATURE: A primer from the flanking DNA of 5' to gene 1 of P-
39 in B. burgdorferi.
(A) NAME/KEY:
(B) LOCATION:
(C) IDENTIFICATION METHOD:
(D) OTHER INFORMATION:
(x) PUBLICATION INFORMATION:
(A) AUTHORS:
(B) TITLE:
(C) JOURNAL:
(D) VOLUME:
(E) ISSUE:
(F) PAGES:
(G) DATE:
(H) DOCUMENT NUMBER:
(I) FILING DATE:
(J) PUBLICATION DATE:
(K) RELEVANT RESIDUES:
(xi) SEQUENCE DESCRIPTION: SEQ ID NO: 4:
ATG AAT AAA ATA TTG TTG 18

(2) INFORMATION FOR SEQ ID NO: 5:
(i) SEQUENCE CHARACTERISTICS:
(A) LENGTH: 18
(B) TYPE: Nucleic Acid
(C) STRANDEDNESS: Single-Stranded
(D) TOPOLOGY: Linear
(ii) MOLECULE TYPE: Synthetic DNA
(A) DESCRIPTION: A primer from within the
insertion sequence of genes 1 and 2 of P-39 in B. burgdorferi.
(iii) HYPOTHETICAL:
(iv) ANTI-SENSE:
(v) FRAGMENT TYPE:

(vi) ORIGINAL SOURCE:
(A) ORGANISM: Borrelia burgdorferi
(B) STRAIN: Sh-2-82
(C) INDIVIDUAL ISOLATE: Sh-2-82, P-6
(D) DEVELOPMENTAL STAGE:
(E) HAPLOTYPE:
(F) TISSUE TYPE:
(G) CELL TYPE:
(H) CELL LINE:
(I) ORGANELLE:
(vii) IMMEDIATE SOURCE:
(A) LIBRARY:
(B) CLONE: pSPR33
(viii) POSITION IN GENOME:
(A) CHROMOSOME/SEGMENT:
(B) MAP POSITION:
(C) UNITS:
(ix) FEATURE: A primer from within the insertion
sequence of genes 1 and 2 of P-39 in B. burgdorferi.
(A) NAME/KEY:
(B) LOCATION:
(C) IDENTIFICATION METHOD:
(D) OTHER INFORMATION:
(x) PUBLICATION INFORMATION:
(A) AUTHORS:
(B) TITLE:
(C) JOURNAL:
(D) VOLUME:
(E) ISSUE:
(F) PAGES:
(G) DATE:
(H) DOCUMENT NUMBER:
(I) FILING DATE:
(J) PUBLICATION DATE:
(K) RELEVANT RESIDUES:
(xi) SEQUENCE DESCRIPTION: SEQ ID NO: 5:
AAT AAA TTC TTT AAG AAA 18

(2) INFORMATION FOR SEQ ID NO: 6:
(i) SEQUENCE CHARACTERISTICS:
(A) LENGTH: 18
(B) TYPE: Nucleic Acid
(C) STRANDEDNESS: Single-Stranded
(D) TOPOLOGY: Linear
(ii) MOLECULE TYPE: Synthetic DNA
(A) DESCRIPTION: A primer from within the
insertion sequence of genes 1 and 2 of P-39 in B. burgdorferi.
(iii) HYPOTHETICAL:
(iv) ANTI-SENSE:
(v) FRAGMENT TYPE:
(vi) ORIGINAL SOURCE:
(A) ORGANISM: Borrelia burgdorferi
(B) STRAIN: Sh-2-82
(C) INDIVIDUAL ISOLATE: Sh-2-82, P-6
(D) DEVELOPMENTAL STAGE:
(E) HAPLOTYPE:
(F) TISSUE TYPE:
(G) CELL TYPE:
(H) CELL LINE:
(I) ORGANELLE:
(vii) IMMEDIATE SOURCE:
(A) LIBRARY:
(B) CLONE: pSPR33
(viii) POSITION IN GENOME:
(A) CHROMOSOME/SEGMENT:
(B) MAP POSITION:
(C) UNITS:
(ix) FEATURE: A primer from within the insertion
sequence of genes 1 and 2 of P-39 in B. burgdorferi.
(A) NAME/KEY:
(B) LOCATION:
(C) IDENTIFICATION METHOD:
(D) OTHER INFORMATION:
(x) PUBLICATION INFORMATION:
(A) AUTHORS:
(B) TITLE:

(C) JOURNAL:
(D) VOLUME:
(E) ISSUE:
(F) PAGES:
(G) DATE:
(H) DOCUMENT NUMBER:
(I) FILING DATE:
(J) PUBLICATION DATE:
(K) RELEVANT RESIDUES:
(xi) SEQUENCE DESCRIPTION: SEQ ID NO: 6:
ATG AGA ATT GTA ATT TTT 18
(2) INFORMATION FOR SEQ ID NO: 7:
(i) SEQUENCE CHARACTERISTICS:
(A) LENGTH: 18
(B) TYPE: Nucleic Acid
(C) STRANDEDNESS: Single-Stranded
(D) TOPOLOGY: Linear
(ii) MOLECULE TYPE: Synthetic DNA
(A) DESCRIPTION: A primer from within the
insertion sequence of genes 1 and 2 of P-39 in B. burgdorferi.
(iii) HYPOTHETICAL:
(iv) ANTI-SENSE:
(v) FRAGMENT TYPE:
(vi) ORIGINAL SOURCE:
(A) ORGANISM: Borrelia burgdorferi
(B) STRAIN: Sh-2-82
(C) INDIVIDUAL ISOLATE: Sh-2-82, P-6
(D) DEVELOPMENTAL STAGE:
(E) HAPLOTYPE:
(F) TISSUE TYPE:
(G) CELL TYPE:
(H) CELL LINE:
(I) ORGANELLE:
(vii) IMMEDIATE SOURCE:
(A) LIBRARY:
(B) CLONE: pSPR33

(viii) POSITION IN GENOME:
(A) CHROMOSOME/SEGMENT:
(B) MAP POSITION:
(C) UNITS:
(ix) FEATURE: A primer from within the insertion
sequence of 5' to gene 2 of P39 in B. Burgdorferi.
(A) NAME/KEY:
(B) LOCATION:
(C) IDENTIFICATION METHOD:
(D) OTHER INFORMATION:
(x) PUBLICATION INFORMATION:
(A) AUTHORS:
(B) TITLE:
(C) JOURNAL:
(D) VOLUME:
(E) ISSUE:
(F) PAGES:
(G) DATE:
(H) DOCUMENT NUMBER:
(I) FILING DATE:
(J) PUBLICATION DATE:
(K) RELEVANT RESIDUES:
(xi) SEQUENCE DESCRIPTION: SEQ ID NO: 7:
TAA ATT TAA TAT TTG TTT 18

## Description

### FIELD OF THE INVENTION

The present invention relates to antigenic *Borrelia burgdorferi* proteins and their encoding DNA. In particular, the present invention relates to two 39 kilodalton (kDa) *Borrelia burgdorferi* proteins which react with Lyme borreliosis serum and a 28 kDa *Borrelia burgdorferi* protein which reacts with Lyme borreliosis serum.

### BACKGROUND INFORMATION

Lyme borreliosis in humans is a multisystemic disorder caused by infection with the tick-borne spirochete, *Borrelia burgdorferi*, (Burgdorfer et al. 1982. Science 216:1317-1319; Johnson et al. 1984. Int. J. Syst. Bacteriol. 34:496-497; and Steere et al. 1983. N. Engl. J. Med. 308:733-740). Since the first epidemiological investigations of this disease in south-central Connecticut (Steere et al. 1977. Ann. Intern. Med. 86:685-698 and Steere et al. 1977. Arthritis. Rheum. 20:7-17), human cases of Lyme borreliosis have now been identified in 43 states of the United States (Centers for Disease Control 1989, Lyme Disease - United States, 1987 and 1988. MMWR 38:668-672), five provinces of Canada, (Centers for Disease Control 1989, Lyme disease - Canada. MMWR 38:677-678), numerous countries throughout Europe and Asia (Ai et al. 1988. Ann. NY Acad. Sci. 539:302-313; Dekonenko et al. 1988. J. Infect. Dis. 158:748-753; and Schmid. 1985. Rev. Infect. Dis. 7:41-50), and possibly restricted foci in Australia (Stewart et al. 1982. Med. J. Australia 1:139) and Africa (Haberberger et al. 1989. Trans. R. Soc. Trop. Med. Hyg. 83:556 and Stanek et al. 1986. Zentralbl. Bakteriol. Mikrobio. Hyg. [A] 263:491-495). Between 1982-1988, reports of 13,825 cases of Lyme borreliosis were received by the Centers for Disease Control from all 50 states of the United States, (Centers for Disease Control 1989, Lyme Disease - United States, 1987 and 1988. MMWR 38:668-672), making this disease the most prevalent arthropod-borne infection in the country.

With the dramatic increase in awareness, prevalence, and geographical distribution of Lyme borreliosis, a tremendous new demand has been placed on clinical laboratories for serological confirmation of cases, (Magnarelli. 1989. J. Am. Med. Assoc. 262:3464-3465 and Schwartz et al. 1989. J. Am. Med. Assoc. 262:3431-3434) or to rule out this disease in differential diagnoses. However, many potential problems exist with the currently available serological tests for Lyme borreliosis, which may result in either false positive or false negative results (Magnarelli 1989. J. Am. Med. Assoc. 262:3464-3465). Some studies have focused on using flagellar protein of *B. burgdorferi* to increase the sensitivity of serological tests (Hansen et al. 1989. J. Clin. Microbiol 27:545-551 and Hansen et al. 1988. J. Clin. Microbiol 26:338-346) because earlier studies demonstrated that it appeared to be the 41 kilodalton (kDa) flagellar subunit (flagellin) of the spirochete that generated the earliest antibody response in infected humans (Barbour et al. 1983. J. Clin. Invest. 72:504-515; Coleman et al. 1987. J. Infect. Dis. 155:756-765; and Grodzicki et al. 1988. J. Infect. Dis. 157:790-797). One of two potential problems with using flagellar protein, however, is that flagella of other *Borrelia* species share epitopes common to the flagella of *B. burgdorferi* (Barbour et al. 1986. Infect. Immun. 52:549-544). Secondly, in most studies that have screened human sera by immunoblot analysis (Barbour. 1984. Yale J. Biol. Med. 57:581-586; Barbour et al. 1983. J. Clin. Invest. 72:504-515; Coleman et al. 1987. J. Infect. Dis. 155:756-765; Craft et al. 1986. J. Clin. Invest. 78:934-939; and Nadal et al. 1989. Pediatr. Res. 26:377-382), antibodies binding the protein with an apparent migration of 41 kDa have been assumed, but not proven, to be flagellin.

Thus, it is clear that a need exists for a method of detecting Lyme borreliosis disease in mammals. The present invention provides such a method.

Howe et al, Infection and Immunity 54(1):207-212 (1986), disclose genes encoding two outer membrane proteins of the Lyme disease agent Borrelia burgdorferi.

Karlson et al, Serodiagnosis and Immunotherapy in Infectious Disease 2:375-386 (1988), and Nadal et al, Pedatric Research 26(4):377-382, disclose proteins of 39 kD from Borrelia burgdorferi, identified by means of immunoblotting with sera from patients with Lyme disease.

### SUMMARY OF THE INVENTION

According to one aspect of this invention, a novel protein available from Borrelia burgdorferi, having a molecular weight of about 39 kilodalton as determined by SDS-PAGE, or a portion thereof, having specific reactivity with mammalian Lyme borreliosis serum but not with sera from patients suffering other spirochete-based diseases, is expressed from a DNA fragment comprising the nucleotide sequence shown in any of SEQ ID Nos. 1, 2 and 3. This protein is sometimes described herein as P39; reference is also made to an analogous protein described as P28 (which is not part of this invention).

Further, a novel DNA fragment encodes a protein as defined above. It may be incorporated into a vector, and used to transform a host cell which itself can be used to express the protein.

The protein may be bound to a solid support, to form a complex. A surface coated with the protein can be used in a bioassay for the diagnosis of Lyme borreliosis disease, by contacting the coated surface with serum from a mammal suspected of having Lyme disease, and detecting the presence or absence of a complex formed between the protein and antibodies, in the serum, specific for the protein.

Another aspect of the invention comprises antibodies specific for the novel protein. A novel complex comprises the antibody bound to a solid support. A surface coated with the antibody can be used in a bioassay for the diagnosis of Lyme borreliosis disease, by contacting the coated surface with serum from a mammal suspected of having Lyme disease, and detecting the presence or absence of a complex formed between the antibody and the proteins present in the serum.

A diagnostic kit according to the invention comprises a novel protein and ancillary reagents suitable for use in detecting the presence of antibodies to the protein. Further, a method of screening a drug for anti-Lyme borreliosis disease activity comprises contacting the drug with cells contacted with *Borrelia burgdorferi* under conditions such that inhibition of anti-Lyme activity can be effected.

Various other objects and advantages of the present invention will become obvious from the drawings and the following description of the invention.

All publications mentioned herein are hereby incorporated by reference.

### BRIEF DESCRIPTION OF THE DRAWINGS

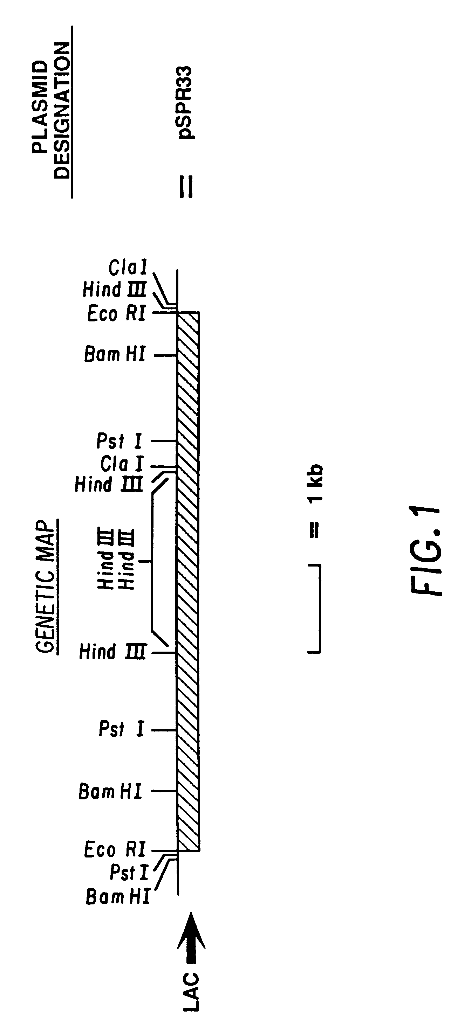

FIGURE 1 shows a genetic map of pSPR33. Spirochete DNA is denoted as a striped block and the arrow indicates the direction that the *Lac* promoter is transcribed. There were no restriction sites within the spirochete *Eco*RI fragment for *Acc*I, *Kpn*I, *Xba*I, *Xho*I or *Sma*I.

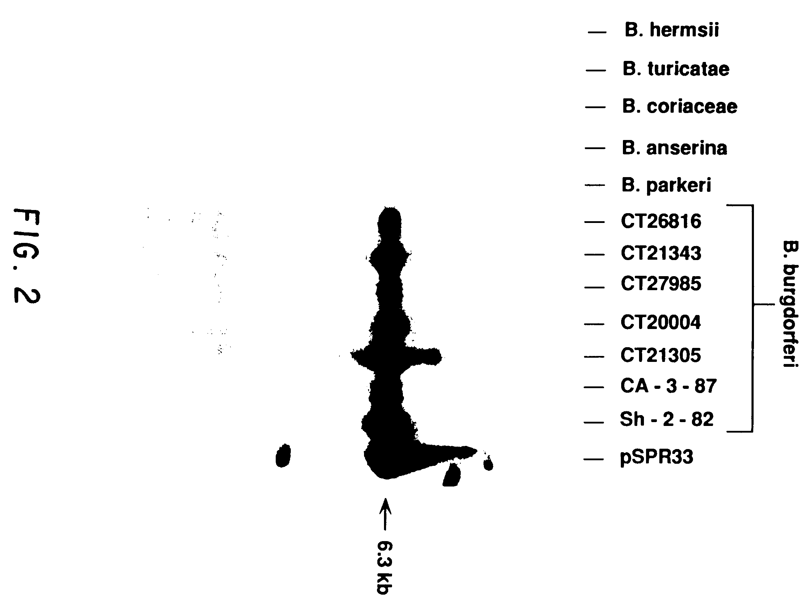

FIGURE 2 is an autoradiograph showing hybridization of ³²P-labeled insert DNA from pSPR33 with total DNA digested with *Eco*RI from 7 isolates of *B. burgdorferi* and 5 other *Borrelia* species. The right lane contained pSPR33 (pSPR33) digested with *Eco*RI. Linear molecular weight markers (Kb) are indicated on the right of the panel.

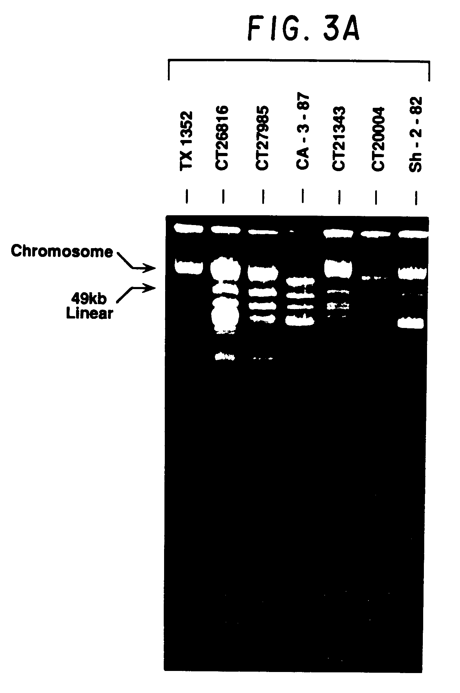

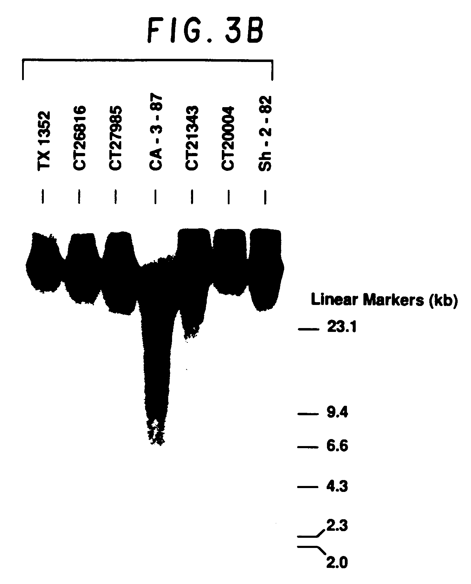

FIGURE 3 is an ethidium bromide stained gel of undigested total DNA from 7 isolates of *B. burgdorferi* (panel A) and an autoradiograph of the same gel after blotting to nitrocellulose and hybridization with the ³²P-labeled 6.3 Kb *Eco*RI fragment from pSPR33 (panel B). Note the strong hybridization signal associated with the chromosomal band.

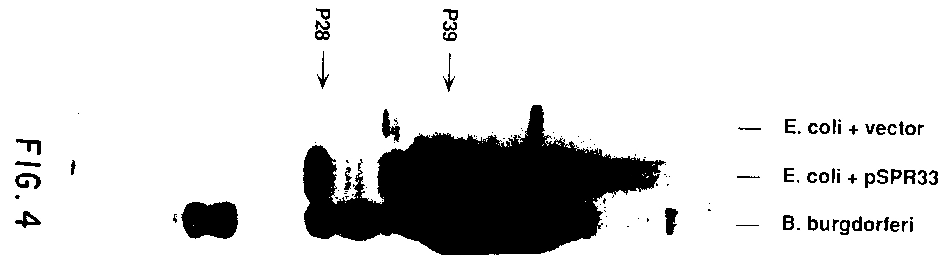

FIGURE 4 shows immunoblot analysis of proteins expressed by pSPR33. Whole-cell lysates of *B. burgdorferi* strain Sh-2-82, *E. coli* carrying pSPR33 (*E. coli* + pSPR33) and *E. coli* carrying only vector (*E. coli* + vector) were immunoblotted with the human Lyme borreliosis serum used to screen the DNA library of *B. burgdorferi*.

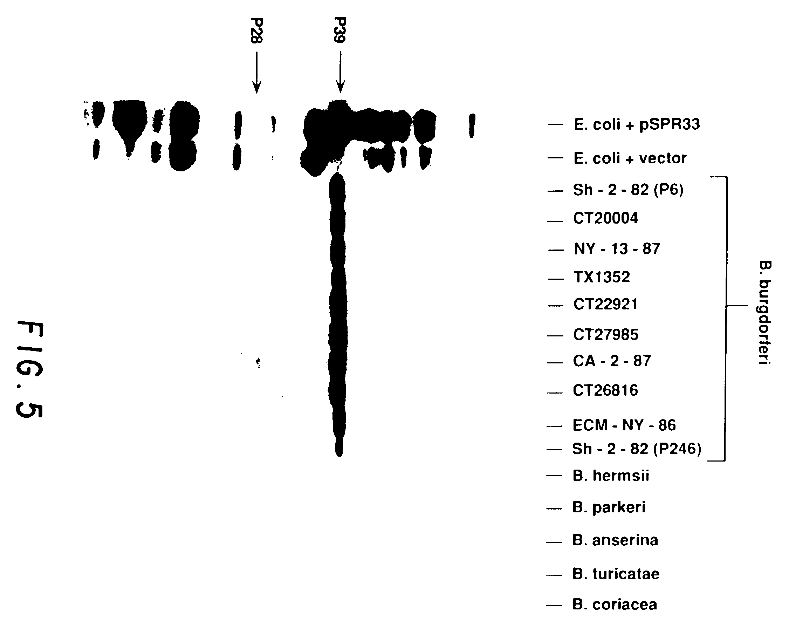

FIGURE 5 demonstrates the specificity of P28 and P39 expression in *B. burgdorferi*. Whole-cell lysates of different *B. burgdorferi* strains (including low (P6) and high (P246) *in vitro* passages of strain Sh-2-82) and isolates representing 5 additional *Borrelia* species were immunoblotted with anti-pSPR33 serum. Lysates of *E. coli* that express P28 and P39 (*E. coli* + pSPR33) and that do not (*E. coli* + vector) were also immunoblotted as positive and negative controls respectively. Not all of the 20 *B. burgdorferi* isolates tested are shown (see Table 1).

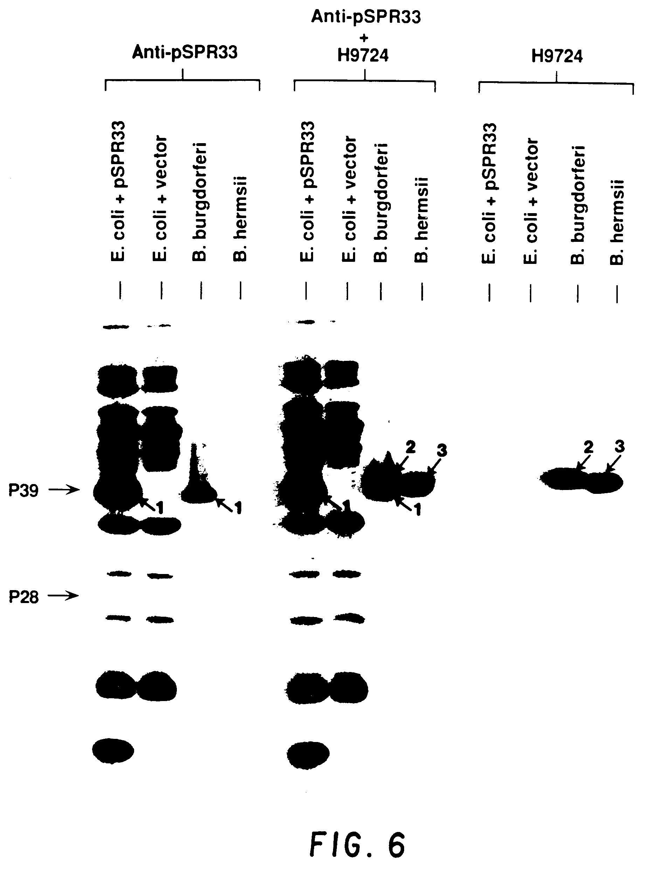

FIGURE 6 comprises the reactivity of anti-pSPR33 and monoclonal antibody H9724. Components in whole cell lysates of *E. coli* plus pSPR33, *E. coli* plus vector only, *B. burgdorferi* strain Sh-2-82 and *B.hermsii* strain FRG were separated by SDS-PAGE and were incubated with anti-pSPR33, anti-pSPR33 plus H9724, or H9724. P39 (arrow 1); 41 kDa flagellin from *B. burgdorferi* (arrow 2); 39 kDa flagellin from *B. hermsii* (arrow 3).

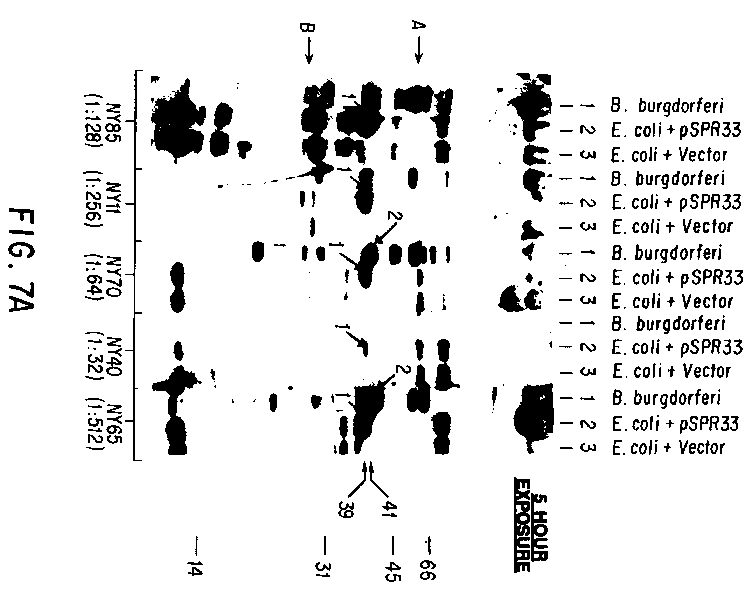

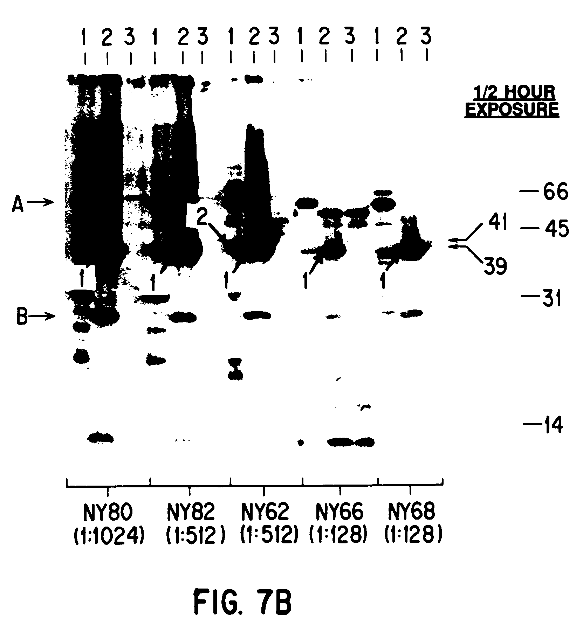

FIGURE 7 shows immunoblot analysis of 10 human Lyme borreliosis sera and their reactivity with P28 and P39. Whole-cell lysates of *B. burgdorferi* strain Sh-2-82 (lane 1), *E. coli* carrying pSPR33 (lane 2) and *E. coli* carrying only vector (lane 3) were immunoblotted with human Lyme borreliosis sera (NY). IFA Lyme borreliosis titers for each human serum are indicated below their designations. Autoradiographs exposed for 5 hr. (panel A) represented sera having weaker reactivity than those exposed for 1/2 hr. (panel B). Arrows denote P39 (arrow 1) and a 41 kDa antigen (arrow 2). Band B corresponds to the position of P28 and band A is an 58-65 kDa antigen that bound all sera that reacted with P39. Molecular mass markers (kDa) are indicated on the right of each panel.

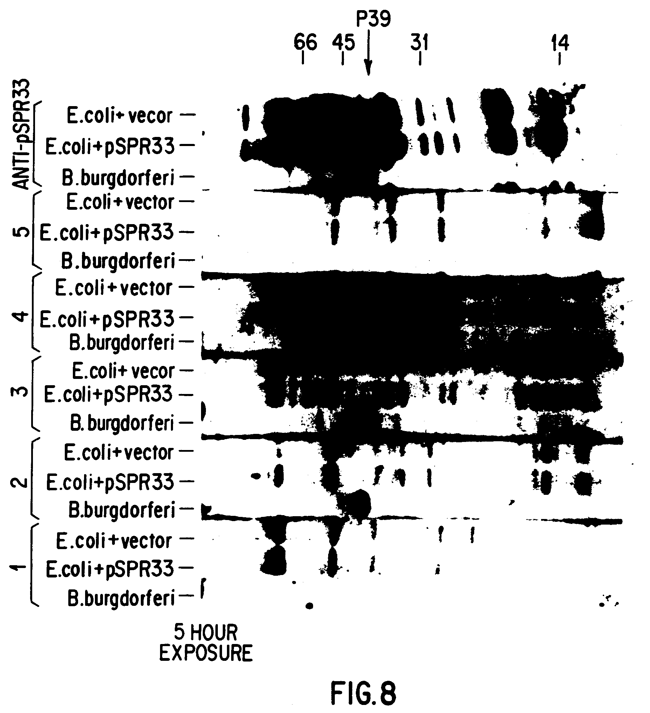

FIGURE 8 shows immunoblot analysis of syphilitic sera. Whole-cell lysates of *B. burgdorferi* strain Sh-2-82, *E. coli* carrying pSPR33 and *E. coli* carrying only vector were immunoblotted with 5 syphilitic sera (1 to 5) or anti-pSPR33 (anti-pSPR33). Molecular mass markers are indicated on the right. Note absence of P39 in pSPR33 lanes reacted with syphilitic sera which contrasts with a strongly reactive 41 kDa antigen in three of the five *B. burgdorferi* lanes.

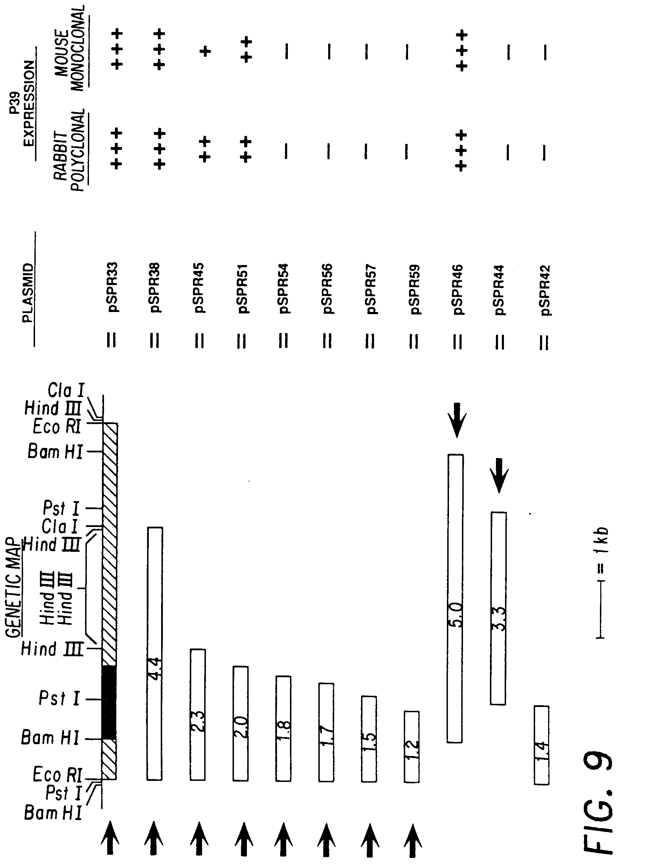

FIGURE 9 shows a restriction endonuclease map and expression data for the P39 locus of *Borrelia burgdorferi*. Subclones and deletion variants of plasmid pSPR33 (pSPR38, pSPR45, pSPR51, pSPR54, pSPR56, pSPR57, pSPR59, pSPR38, pSPR45, pSPR51, pSPR54, pSPR56, pSPR57, pSPR59, pSPR46, pSPR44 and pSPR42) are indicated as open bars.

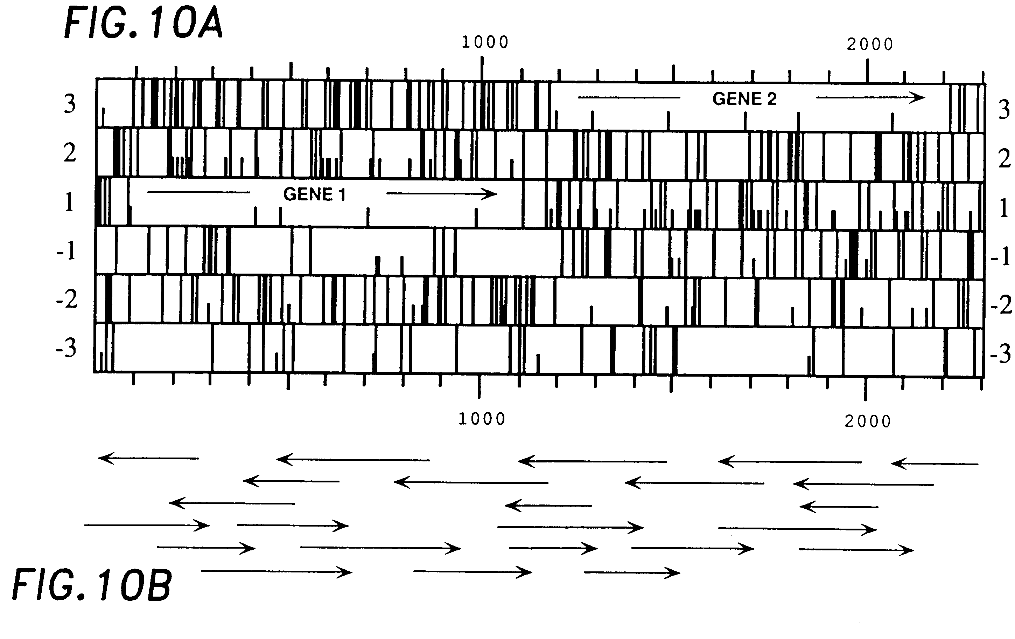

FIGURES 10a and 10b show a map of the open reading frames of gene 1 and gene 2 encoding the P39 α and P39 β antigens, respectively, of *Borrelia burgdorferi*. Figure 10a shows the frames clear of termination sites (complete vertical lines). Figure 10b shows primer sites with overlapping sequences used to determine nucleotide sequences of both strands of DNA.

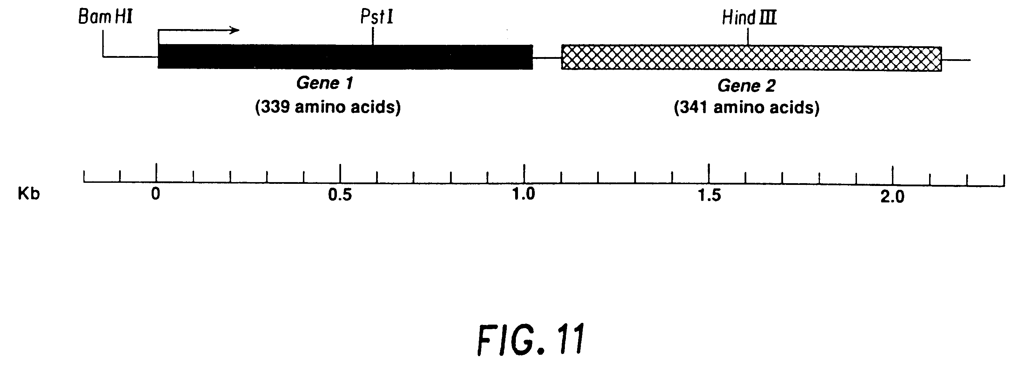

FIGURE 11 shows a genetic map of the P39 operon of *Borrelia burgdorferi* including the position of genes 1 and 2, the number of deduced amino acid, and the direction of transcription.

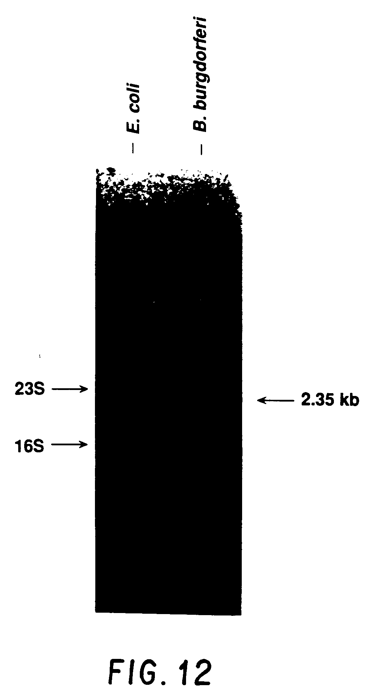

FIGURE 12 shows northern blot of *Borrelia burgdorferi* RNA probed with pSPR33 showing a 2.35 kb RNA transcript of the appropriate size for the single transcriptional unit for genes 1 and 2.

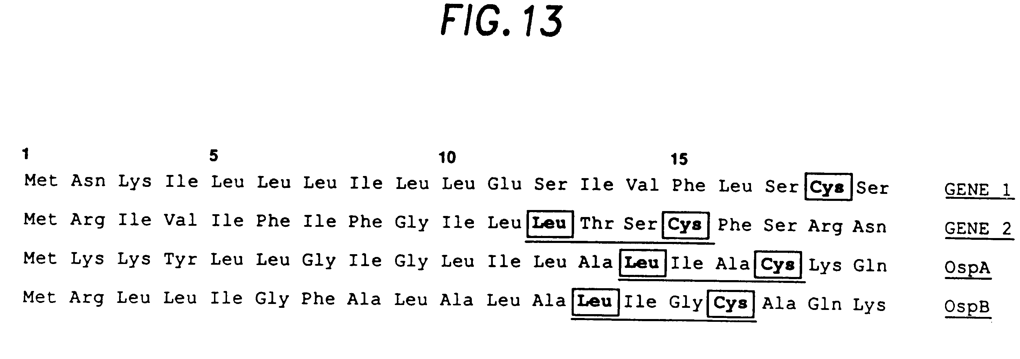

FIGURE 13 shows the deduced amino-terminal ends of P39 α (gene 1) and P39 β (gene 2), and the major outer surface proteins (Osp) A and B of *Borrelia burgdorferi*.

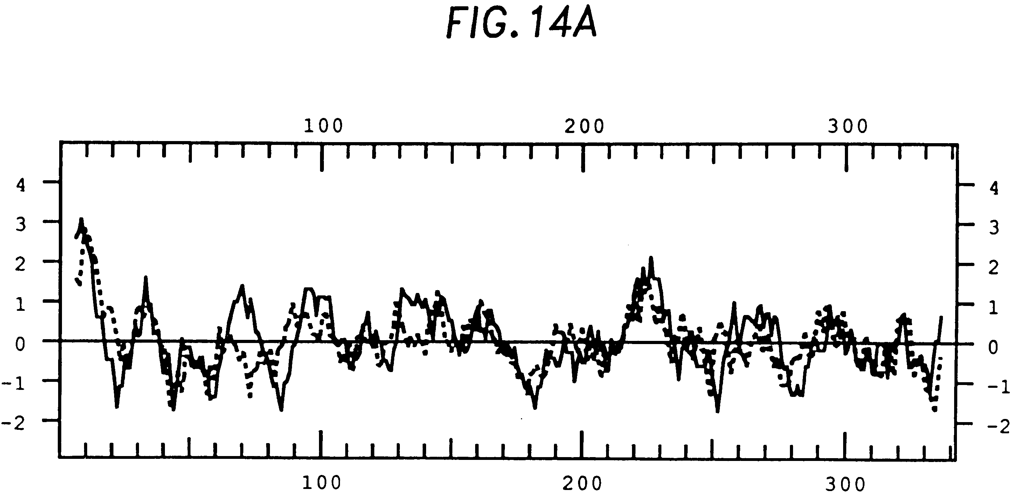

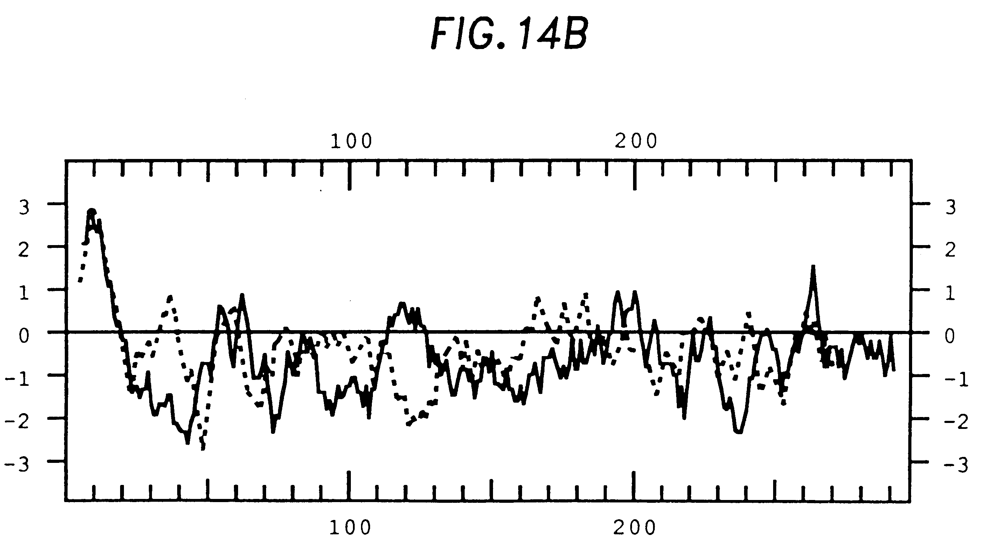

FIGURE 14 shows hydrophobicity plots of the deduced amino acid sequences of P39 α (dotted line) and P39 β (solid line) (Panel A) and OspA (dotted line) and OspB (solid line) (Panel B) of *Borrelia burgdorferi* (+ values show hydrophilic regions and - values show hydrophobic regions of the proteins).

### DETAILED DESCRIPTION OF THE INVENTION

This invention relates, in part, to *Borrelia burgdorferi* antigenic proteins and their encoding DNA. A principal embodiment of this aspect of the present invention relates to two antigenic *Borrelia burgdorferi* proteins. Two proteins are characterized by a molecular weight of about 39 kDa (designated 39α and 39β) as determined by SDS-PAGE and reactivity with human Lyme borreliosis serum. The present invention also relates to unique portions of the above proteins wherein a unique portion consists of at least 5 (or 6) amino acids.

The 39 kDa proteins are substantially free of proteins with which they are normally associated. A substantially pure form of the proteins of the present invention can be obtained by one skilled in the art using standard methodologies for protein purification without undue experimentation. The present invention also relates to peptide fragments of the 39 kDa protein. Alternatively, the proteins and peptides of the invention can be chemically synthesized using known methods.

The present invention also relates to a DNA fragment encoding all, or a unique portion, of the 39 kDa *B. burgdorferi* proteins of the present invention. A principal embodiment of this aspect of the invention relates to the 6.3 kilobase pair *Eco*RI fragment obtained from a DNA library of *B. burgdorferi* DNA which encodes the 39 kDa antigenic proteins.

The present invention also relates to a DNA fragment encoding all, or a unique portion, of the 39 kDa α *B. burgdorferi* protein or the 39 kDa β *B. burgdorferi* protein.

The present invention further relates to a recombinant DNA molecule and to a host cell transformed therewith. Using standard methodology well known in the art, a recombinant DNA molecule comprising a vector and a DNA fragment encoding both the 39 kDa proteins of this invention, either of the 39 kDa proteins can be constructed using methods known in the art without undue experimentation. The DNA fragment can be isolated from *B. burgdorferi*, and it can take the form of a cDNA clone produced using methods well known to those skilled in the art or it can be produced by polymerase chain reaction. Possible vectors for use in the present invention include, but are not limited to, λZAPII, pUC8 or preferably high frequency expression vectors such as pBluescript II SK, pNH8a. The host cell can be prokaryotic (such as bacterial), lower eukaryotic (such as fungal, including yeast) or higher eukaryotic (such as mammalian).

The present invention further relates to antibodies specific for the 39 kDa *B. burgdorferi* proteins of the present invention. One skilled in the art using standard methodology can raise monoclonal antibodies and polyclonal antibodies to the 39 kDa proteins, or a unique portion thereof. This is exemplified by the anti-pSPR33 rabbit antiserum (see Example 2 below).

The present invention also relates to a vaccine for use in mammals against Lyme borreliosis disease. In one embodiment of this aspect of this invention, as is customary for vaccines, either of the 39 kDa proteins of the present invention can be delivered to a mammal in a pharmacologically acceptable vehicle. As one skilled in the art will understand, it is not necessary to use the entire protein. A unique portion of the protein (for example, a synthetic polypeptide corresponding to a portion of the 39 kDa proteins) can be used. Vaccines of the present invention can include effective amounts of immunological adjuvants known to enhance an immune response. The protein or polypeptide is present in the vaccine in an amount sufficient to induce an immune response against the antigenic protein and thus to protect against Lyme borreliosis infection. Protective antibodies are usually best elicited by a series of 2-3 doses given about 2 to 3 weeks apart. The series can be repeated when circulating antibodies concentration in the patient drops.

The present invention further relates to diagnostic assays for use in human and veterinary medicine. For diagnosis of Lyme borreliosis disease, the presence of antibodies to the 39 kDa proteins in mammalian serum is determined. Many types of tests, as one skilled in the art will recognized, can be used for detection. Such tests include, but are not limited to, IFA, RIA, RIST, ELISA, aggulination and hemagglutination. The diagnostic assays can be performed using standard protocols such as those described by Magnarelli et al. 1984. J. Clin. Microbiol. 20:181-184; Craft et al. 1984. J. Infect. Dis. 149:789-795; Enguall et al. 1971. Immunochemistry 8:871-874; and Russell et al. 1984. J. Infect. Dis. 149:465-470.

Specifically, a diagnostic assay of the present invention can be constructed by coating on a surface (ie. a solid support) for example, a microtitration plate or a membrane (eg. nitrocellulose membrane), all or a unique portion of the 39 kDa proteins (natural or synthetic), and contacting with the serum from a patient suspected of having Lyme borreliosis disease. The presence of a resulting complex formed between the surface and antibodies specific therefore in the serum can be detected by any of the known methods common in art, such as fluorescent antibody spectroscopy or colorimetry.

In another embodiment of the diagnostic assay of the present invention, all or a unique portion of the 39 kDa proteins is bound to an inert particle of, for example, bentonite or polystyrene latex. The particles are mixed with serum from a patient in, for example, a well of a plastic agglutination tray. The presence or absence of antibodies in the patient's serum is determined by observing the settling pattern of the particles in the well.

In a further embodiment of the diagnostic assay of the present invention, the presence or absence of the 39 kDa proteins in a serum sample is detected. Antibodies specific for the 39 kDa proteins, or a unique portion thereof can be coated on to a solid surface such as a plastic and contacted with the serum sample. After washing, the presence or absence of the protein from the serum bound to the fixed antibodies is detected by addition of a labeled (e.g. fluorescently labeled) antibody specific for the 39 kDa proteins.

One skilled in the art will appreciate that the invention includes the use of competition type assays in detecting in a sample the antigens and antibodies to which this invention relates.

The present invention further relates to screening for anti-Lyme borreliosis disease drugs. In one embodiment potential anti-Lyme borreliosis disease drugs are tested for their ability to inhibit expression of the 39 kDa proteins in cells contacted with the *B. burgdorferi*. The presence or absence of the 39 kDa proteins in exposed cells treated with test drugs can be determined by any of the standard diagnostic assays mentioned above.

The present invention further relates to DNA fragments containing the nucleotide sequence as shown in Seq. Id No. 1-3, or mutants thereof, to recombinant molecules containing the DNA fragments and host cells transformed with the recombinant molecules. Using standard methodology well known in the art, a recombinant DNA molecule comprising a vector and the DNA fragments of this invention can be constructed using methods known in the art without undue experimentation. The DNA fragments can be isolated from *B. burgdorferi* or can be produced by a polymerase chain reaction. Possible vectors for use in the present invention include but are not limited to, pUC, pBluescript or pBR322. The host cell can be prokaryotic (such as bacterial), lower eukaryotic (such as fungal, including yeast) or higher eukaryotic (such as mammalian).

The present invention further relates to methods of producing recombinant *Borrelia burgdorferi* 39 kDa proteins comprising culturing the aforementioned host cells in a manner allowing expression of the proteins and isolating the proteins from the host cells. Methodology utilize to produce recombinant *B. burgdorferi* proteins are well within the skill of an ordinary artisan.

### EXAMPLES

The following organisms and materials were used throughout the Examples.

Bacterial strains. *B. burgdorferi* strains used (See Table 1 below) have been previously described or were kindly provided by Dr. John Anderson (Connecticut Agriculture Experiment station, New Haven, Conn.), Dr. Alan MacDonald (Southampton Hospital, Long Island, N.Y.), and Ms. Glenna Teltow and Ms. Julie Rawlings (Medical Entomology Section, Bureau of Laboratories, Texas Department of Health, Austin, Tex.). The five strains representing *B. hermsii* (HS1), *B. coriaceae* (Co53), *B. parkeri*, *B. turicatae* and *B. anserina* have been described previously (Schwan et al. 1989. J. Clin. Microbiol. 27:1734-1738). Borrelia organisms were cultured at 32°C in BSK-II medium as previously described (Barbour. 1984. Yale J. Biol. Med. 57:581-586).

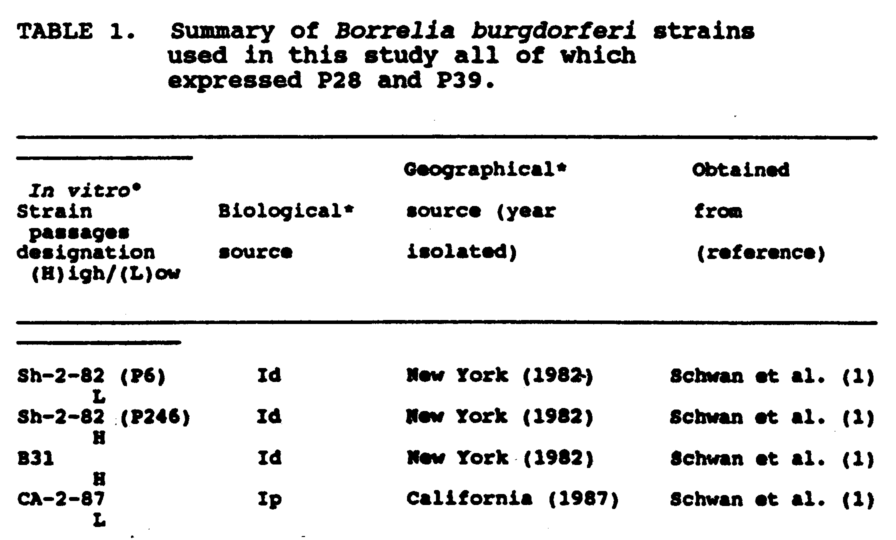

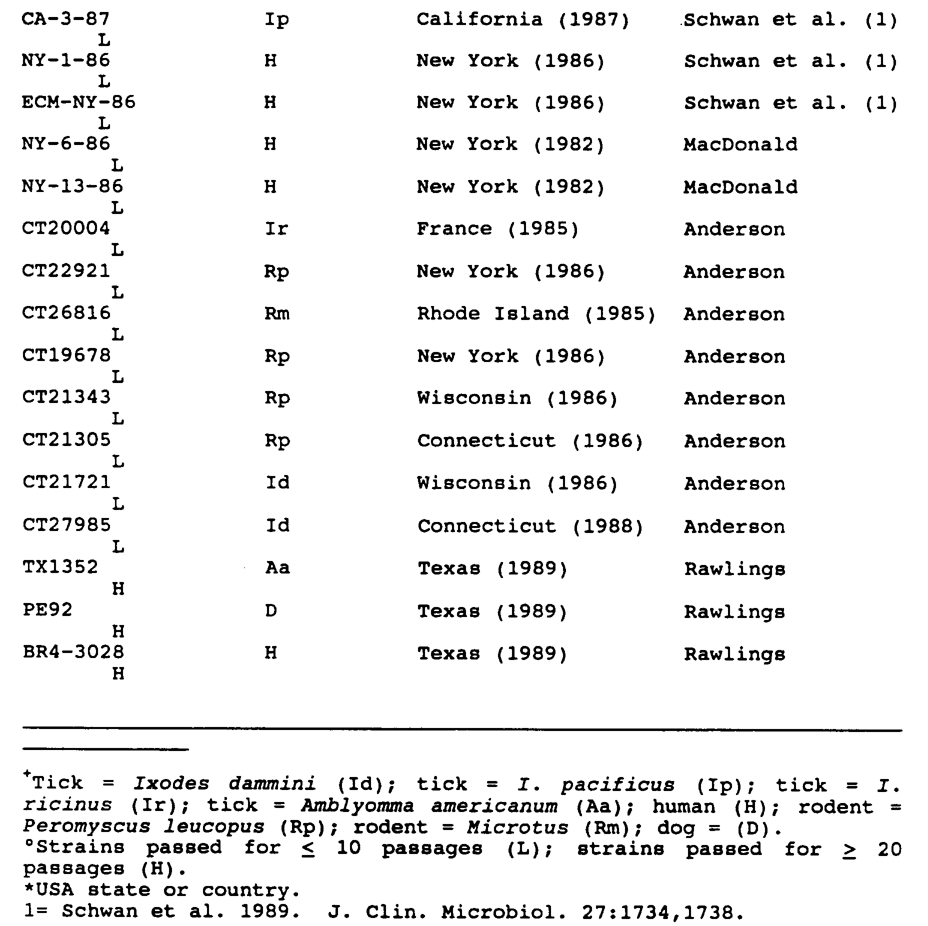

Human syphilitic sera were kindly provided by Dr. Wayne Hogefre and Ms. Jane Markley (Hillcrest Biologicals, Cypress, Calif.), amyotrophic lateral sclerosis (ALS) sera were provided by Dr. Jeffrey Smith (Mount Sinai Medical Center, ALS Clinic, New York, NY.) and Dr. Alan MacDonald (Southampton Hospital, Long Island, N.Y.), and relapsing fever sera were collected from patients from Oregon and Washington. Normal sera were obtained from staff and laboratory personnel at Rocky Mountain Laboratories. Human Lyme borreliosis sera were provided by Dr. Alan MacDonald and were collected from patients clinically diagnosed with Lyme borreliosis from Long Island, New York.

*Escherichia coli* carrying the plasmid pSPR33 (see below) were deposited on February 28, 1990 at the American Type Culture Collection 12301 Parklawn Drive, Rockville, Maryland 20852. The accession number of the organism is 68243. The deposits shall be viably maintaining, replacing it if it becomes non-viable, for the life of the patent, for a period of 30 years from the date of the deposit or for five years from the last date of request or sample of the deposit, whichever is longer and made available to the public upon issuance of a patent from this application, without restriction, and in accordance with the provisions of the law. The Commissioner of Patents and Trademarks, upon request shall have access to the deposit.
Example 1. Cloning and Genetic Analysis of *Borrelia* DNA
To identify *B. burgdorferi* proteins that induce an antibody response during the course of an infection, a DNA library of *B. burgdorferi* containing *Eco*RI fragments was constructed in *E. coli* with the λ expression vector λZAPII.
Total DNA was purified from 500 ml stationary phase borrelial cultures by a modification as previously described (Barbour. 1988. J. Clin. Microbiol. 26:475-478). Cells were recovered by centrifugation, washed in 20 ml of PBS plus 5 mM MgCl₃ and resuspended in 2.4 ml TES (50 mM Tris, pH 8.0; 50 mM EDTA, 15% (w/v) sucrose). Lysozyme was added to a final concentration of 1 mg/ml and then the cell suspension was left on ice for 10 min. Cells were lysed by adding 3 ml TES plus 1% (v/v) sodium deoxycholate and gently mixed for 10 min. at room temperature. Proteinase K (1 mg) was then added and the sample was incubated at 37°C for 1 hr. The DNA suspension was then extracted twice with 1 volume of phenol-chloroform (1:1 (v/v)) and once with chloroform-isoamyl alcohol (24:1 (v/v)). The DNA was ethanol precipitated, washed twice with 70% ethanol and resuspended to a final concentration of 1 mg/ml in TE (10 mM Tris, pH 7.6; 1 mM EDTA).
Total DNA (1 µg) from *B. burgdorferi* strain Sh-2-82 was digested with *Eco*RI, ligated to the dephosphorylated arms of the expression vector λZAPII (Stratagene, La Jolla, Calif.) and packaged according to the manufacturer's directions.
The library was screened for *Borrelia* by immunoblot with a convalescent serum from a human Lyme borreliosis patient from Long Island, New York (1:100) following absorption of plaque proteins to nitrocellulose filters (Maniatis et al. 1982. Molecular Cloning: A Laboratory Manual. Cold spring Harbor Laboratory, Cold Spring Harbor, New York). After blocking for 1 hr. at 25°C in TSE-Tween (50 mM Tris, pH 7.4; 150 mM NaCl; 5 mM EDTA; 0.05% Tween 20), filters were incubated with serum diluted in TSE-Tween with gentle rocking at 25°C for 1 hr. They were then washed for 1 hr. with four changes of TSE-Tween and the bound antibody was detected by incubating the filters with ¹²⁵I-labeled protein A (500,000 cpm/ml) for 1 hr. with rocking. Each filter was then washed four times for 15 min. each with TSE-Tween, dried and autoradiographed with Kodak X-AR5 film.
Positive clones were detected at a frequency of 5%. One recombinant plaque that reacted with human serum was plaque purified and the phagemid carrying the *Borrelia* DNA was excised from the λ sequences with the aid of the helper phage R407 according to the suppliers directions (Stratagene). Excision of the cloned fragment from the purified phage produced the phagemid portion containing a 6.3 kilobase (Kb) *Eco*RI fragment, designated plasmid pSPR33 (Fig. 1). The fragment was isolated from an agarose gel, radiolabeled and shown to hybridize with a similar sized fragment in *Eco*RI digested total DNA from all six North American and one European *B. Burgdorferi* isolates (Fig. 2).
Recombinant plasmid pPSR33 was isolated from *E. coli* for mapping studies from 500 ml cultures and purified as previously described (Simpson et al. 1987. Infect. Immun. 55:2448-2455), except two consecutive dye-buoyant density gradients were preformed (Plasterk et al. 1985. Nature 318:257-263) in a Beckman VTi80 rotary at 70,000 rpm for 4 hr at 18 C. The supercoiled circular plasmid portion was diluted with two volumes of water after the removal of the ethidium bromide and then ethanol precipitated. The plasmid DNA was then resuspended in a minimal volume of TE. Mini-plasmid preparations (Maniatis et al. 1982. Molecular Cloning: A Laboratory Manual. Cold Spring Harbor Laboratory, Cold Spring Harbor, New York) of positive clones were examined by agarose gel electrophoresis after their digestion with *Eco*RI to determine the insert size.
Southern blot analysis of undigested DNA from seven similar isolates, indicated that the 6.3 Kb fragment hybridized strongly with chromosomal DNA (Fig. 3). Undigested total DNA was electrophoresed in 0.4% agarose gels (12 v for 16 hrs). Southern blot procedures including the transfer of DNA from agarose gels to nitrocellose, high stringency hybridization (which permitted 10% basepair mismatch), and autoradiography were as previously described (Spanier et al. 1983. Virology 130:514-522) except that the prehybridization and hybridization buffers and temperatures were as described by Schwan et al. (Schwan et al. 1989. J. Clin. Microbiol. 27:1734-1738).
The DNA probe was recovered from agarose gels using Gene Clean (BIO 101, Inc., La Jolla, Calif.) and labeled with [α-³²P]dCTP (3,000 Ci/mmol) by nick translation according to the directions of the manufacturer (Nick Translation Kit, Bethesda Research Laboratories, Gaithersburg, Md.). The probe was boiled for 4 min. and quenched on ice immediately before adding to the hybridization buffer.
Restriction endonucleases were purchased from Boehringer Mannheim Biochemicals, Indianapolis, Ind., and digestions were performed as recommended by the manufacturer.
The smeared band in agarose-gels that contained heterogenous fragmented DNA and migrated slightly slower than the 49 Kb linear plasmid from strain Sh-2-82 was assume to be chromosomal DNA. Total DNA from 5 additional *Borrelia* species including *B. hermsii*, *B. parkeri*, *B. anserina*, *B. turicatae*, and *B. coriaceae* did not hybridize to the 6.3 Kb fragment (Fig. 2). These data indicate that the pSPR33 insert sequences are chromosomally located and are specific to *B. burgdorferi*.
Example 2. Immunoblot Analysis of Cloned
*B. burgdorferi* Proteins
To identify the specific proteins encoded by pSPR33 that reacted with the human serum used to screen the library, whole-cell lysates of *E. coli* carrying pSPR33 were analyzed by SDS-PAGE and immunoblot.
Rabbit serum prepared against whole-cell lysates of *E. coli* carrying either pSPR33 (anti-pSPR33) or the vector pBluescript SK (anti-*E. coli*) were prepared as follows. Bacterial cells recovered from 16 hr cultures, were washed once and resuspended in phosphate buffered saline (PBS) to a final concentration of 10⁸ cells/ml. The cells were killed by incubating for 30 min. at 56°C and disrupted by sonification on ice (2 min. at an output of 4; Branson Sonifier-Cell Disrupter 185). New Zealand white rabbits were immunized (without adjuvant) intramuscularly with 1.5 ml of the cell sonicate and boosted with the same immunogen at 21 and 42 days after the primary immunization. Sera were collected every 2 weeks thereafter for 4 months, pooled, and 5 ml aliquots absorbed with *E. coli* strain XL1-blue cells (Stratagene) collected from 500 ml cultures and incubated with rotation at 37°C for 4 hr. The bacteria were removed by centrifugation in a VTi80 rotor at 40,000 rpm for 30 min. This process was repeated twice, and absorbed sera were then filtered through a sterile 0.22 µm filter (Millipore Corp., Medford, Mass.) and stored at -20°C. Anti-pSPR33 and anti-*E. coli* sera was used at a dilution of 1:500 and 1:50 respectively. The monoclonal antibodies H5332 (Barbour et al. 1983. Infect. Immun. 41:795-804), H5TS (Barbour et al. 1984. Infect. Immun. 45:94-100), and H9724 (Barbour et al. 1986. Infect. Immun. 52:549-554) were used at a dilution of 1:100.
IFA titers of Lyme borreliosis and relapsing fever sera were determined as previously described (Burgdorfer et al. 1982. Science 216:1317-1319). *B. burgdorferi* strain B31 and *B. hermsii* strain HS1 respectively were used as the antigens in the IFA tests.
Immunoblot analysis of whole-cell lysates were performed essentially as previously described (Schwan et al. 1989. Infect. Immun. 57:3445-3451) except cells were prepared as follows. Cells were recovered from liquid cultures by centrifugation (8,000 xg for 5 min), and resuspended in PBS to give an optical density of 0.2 at 600 nm. Cells from 2 ml of this suspension were recovered by centrifugation and resuspended in 100 µl of distilled water and 50 µl of sample buffer (0.2 M Tris, pH 6.8; 30% (v/v) glycerol; 3% (w/v) SDS; 0.002% (w/v) bromophenol blue). Samples were then boiled for 4 min. and 20 µl loaded onto a 12.5% SDS-PAGE gel. Gel electrophoresis, immunoblotting and detection of bound antibody, using ¹²⁵I protein A, have been described (Schwan et al. 1989. Infect. Immun. 57:3445-3451).
A 28 kDa (P28) and a 39 kDa (P39) antigen in the pSPR33 immunoblot profile, were the most immunoreactive antigens that were not detected in lysates of *E. coli* cells carrying only the vector (Fig. 4). Antisera raised to whole-cell lysates of *E. coli* carrying only the vector (anti-*E. coli* serum) did not react with P28 or P39 at a dilution of 1:50. These two proteins, therefore, are antigenically unrelated to native *E. coli* components and appear to be encoded by the cloned *Borrelia* sequences. P28 and P39 could not be resolved in SDS-PAGE gels stained with either Coomassie blue or silver nitrate because they co-migrate with other, more abundant *E. coli* proteins.
Similar sized proteins to P28 and P39 were detected by immunoblot (Fig. 4) in cell lysates of *B. burgdorferi* strain Sh-2-82, suggesting P28 and P39 are expressed by this strain. To determine if the 28 and 39 kDa *Borrelia* proteins seen in whole cell lysates were identical to the gene products P28 and P39 respectively, antiserum generated to cells carrying pSPR33 (anti-pSPR33 serum) was incubated with Western blotted whole-cell lysates of 1 European and 19 North American *B. burgdorferi* isolates, and compared to a lysate of *E. coli* producing P28 and P39 (Fig. 5). All of the 20 *Borrelia* isolates expressed a 39 kDa protein that co-migrated with P39. A 28 kDa protein was also detected, but considerably less antibody bound this protein than that which bound P39. P39 produced by pSPR33 also reacted with sera from five white-footed mice (*Peromyscus leucopus*) experimentally infected with *B. burgdorferi* strain Sh-2-82, but did not react with the preimmune sera from these animals, or with sera from mice infected only with *E. coli*. Other species of *Borrelia* did not produce detectable amounts of P28, P39 or any other antigenically related proteins under the conditions employed (Fig. 5). Extended exposure (> 24 hours) of autoradiographs revealed weak bands with molecular weights other than 28 kDa and 39 kDa in all *Borrelia* profiles, but these are attributed to non-specific binding. Data, including the fact that DNA from other species of *Borrelia* lacked sequences with close identity to those that encode P28 and P39 (Fig. 2), show that P28 and P39 are proteins specific to *B. burgdorferi*. Furthermore, anti-pSPR33 did not react with the *B. burgdorferi* antigens Osp A (31 kDa), Osp B (34 kDa) or the 41 kDa flagellin, suggesting that these proteins are antigenically unrelated to P28 and P39 (Fig. 5).
To confirm this, it was shown that the monoclonal antibodies H5332, H5TS and H9724 (Fig. 6), which bind specifically to Osp. A (Barbour et al. 1983. Infect. Immun. 41:795-804), Osp B (Barbour et al. 1984. Infect. Immun. 45:94-100) and the flagellin (Barbour et al. 1986. Infect. Immun. 52:549-554) respectively, did not bind to P28 or P39 produced by either pSPR33 or strain Sh-2-82. The specificity of monoclonal antibody H9724 for *Borrelia* flagellin is evident in Figure 6, as this monoclonal only bound a 41 kDa band in the *B. burgdorferi* profile and a 39 kDa band, which corresponds to its flagellin (Barbour et al. 1986. Infect. Immun. 52:549-554), in the *B. hermsii* profile. Furthermore, using electron microscopy and colloidal gold staining, monoclonal antibody H9724 bound to endoflagellin from *B. burgdorferi* whereas anti-pSPR33 did not.
Example 3. Immunoreactivity of Lyme Borreliosis
Sera with Cloned Borrelia Proteins
To test the possibility that P28 and P39 are immunodominant proteins, ninety-four human sera collected from patients clinically diagnosed as having Lyme borreliosis were tested for reactivity with cloned P28 and P39 at a dilution of 1:100. Whole-cell lysates were electrophoresed in SDS-PAGE gels and Western blotted as previously described in the above Examples. The nitrocellulose was cut into equal strips (5 per gel) such that each strip contained lanes for *E. coli* carrying pSPR33, *E. coli* carrying only the vector and *B. burgdorferi* strain Sh-2-82. Each strip was incubated with a different human serum except for one strip from each gel which was incubated with anti-pSPR33 serum. This latter strip served as marker for the positions of P28 and P39. All of 33 sera with IFA titers ≧ 1:256 (100%), 13 of 17 sera (76%) with IFA titers = 1:128, and 14 of 44 sera (32%) with titers ≦ 1:64 reacted with P39 (see Table 2 below). 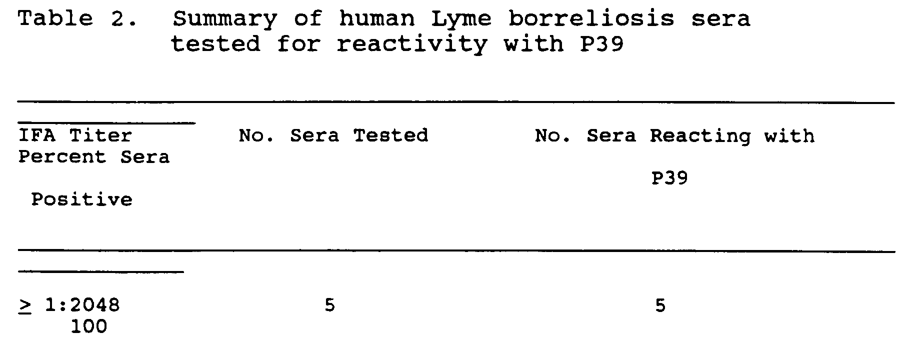 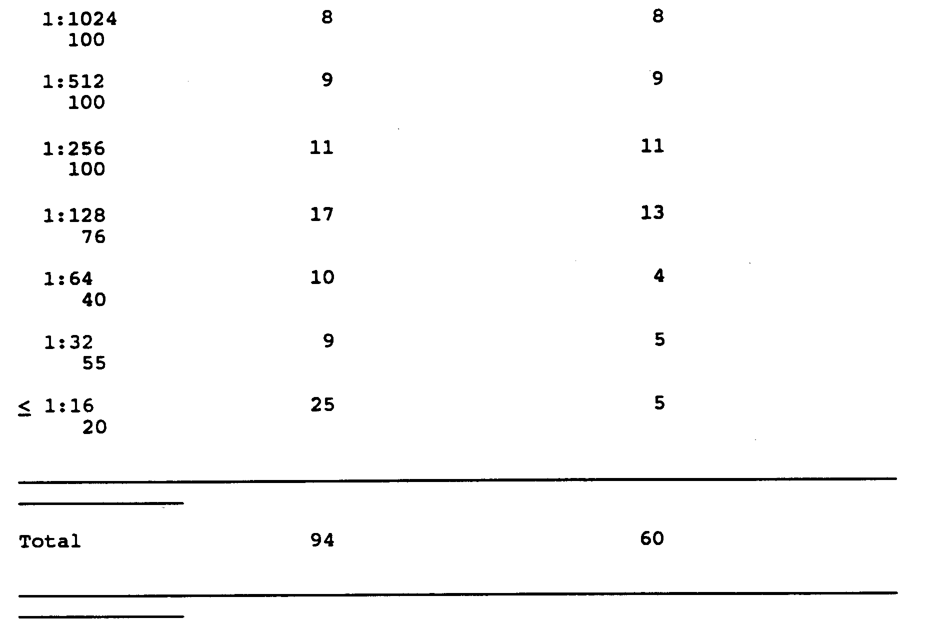
Examples of immunoblots for human sera reacting with P39 (arrow 1) are shown in Figure 7. A strongly reacting 58-65 kDa band was observed in the *B. burgdorferi* profile (Fig. 7, band A) for all sera that reacted with P39, but since anti-pSPR33 serum does not react to a band in this region of the gel (Fig. 5), P39 and the 58-65 kDa protein(s) are presumably unrelated. Although P28 appeared to react strongly to some sera (Fig. 7B, band B), for other, less reactive sera, it was not clear if the sera reacted to P28 or to some other protein. This was because these sera also reacted with co-migrating *E. coli* proteins that were detected with a longer autoradiographic exposure (Fig. 7A, band B). Therefore, although it is not clear to what extent P28 actually reacts with human Lyme borreliosis sera, it appears that antibody to P39 was detected in 100% of all sera that had IFA titers ≧ 1:256. Notably, many sera reactive to P39 did not appear to react with the 41 kDa flagellin (Fig. 7A & 7B). In view of this, antibody to P39 could be mistaken as antibody to the flagellin when testing human sera by immunoblot using whole-cell lysates of *B. burgdorferi*. Because P39 was shown to be specific to *B. burgdorferi* by immunoblot, it is not surprising that control sera, which included sera from 5 ALS patients, 5 syphilitic patients, 5 relapsing fever patients and 10 normal individuals who showed no symptoms of clinical disease, did not react to the cloned P39 protein at a dilution of 1:50 (see Table 3 below). Immunoblot findings for the syphilitic sera are shown in Figure 8. These data suggest that P39 has antigenic specificity for sera collected from patients with Lyme borreliosis. This is despite the fact that both the syphilitic and relapsing fever sera tested had significantly high IFA Lyme borreliosis titers (see Table 3 below), and therefore most likely contained cross-reacting antibodies directed at other *B. burgdorferi* antigens.

**Table 3**

| Summary of IFA titers for control sera that did not react with P39. | | | |
|---|---|---|---|
| Serum description plasma test | Lyme IFA | Relapsing fever IFA | Rapid reagin (1) |
| Syphilitic | | | |
| 1 | 1:128 | 1:256 | 1:128 |
| 2 | 1:256 | 1:1024 | 1:128 |
| 3 | 1:1024 | 1:2048 | 1:128 |
| 4 | 1:512 | 1:1024 | 1:64 |
| 5 | 1:128 | 1:1024 | 1:32 |

| Relapsing fever | | | |
|---|---|---|---|
| 1 | 1:1024 | 1:1024 | --- |
| 2 | 1:32 | 1:512 | --- |
| 3 | 1:128 | 1:512 | --- |
| 4 | 1:64 | 1:512 | --- |
| 5 | 1:64 | 1:1024 | --- |

| ALS | | | |
|---|---|---|---|
| 1 | 1:16 | 1:64 | --- |
| 2, 3, 4 | <1:16 | <1:16 | --- |
| 5 | 1:16 | 1:16 | --- |

| Normal | | | |
|---|---|---|---|
| 1, 2, 3, 4 | <1:16 | 1:16 | --- |
| 5, 6, 7, 8 | <1:16 | <1:16 | --- |
| 9, 10 | <1:16 | 1:32 | --- |
| 1=Portnoy, 1963. Amer. J. Clin. Pathol. 40:473-479 | | | |

The immunodominance of P39 and this antigens' potential to be a virulence factor of *B. burgdorferi* on account of its immune characteristics and association with infectivity, lead to further characterization of the genetic basis for P39 expression.
Example 4. PSPR 33 Subclone and deletion analysis. Eleven subclones were constructed to determine the approximate position of the P39 locus in the 6.3 kb EcoRI insert in the parent construct pSPR33. The endonucleases EcoRI, ClaI, HindIII, BamHI and PstI were used according to the manufacturer (Boehringer Mannheim Biochemicals) to produce various restriction fragments, which were then ligated to the linearized pBluescript cloning vector (Stratagene) cut with the appropriate enzyme or combination of enzymes. A 4.4 kb EcoRI - ClaI fragment was ligated into the vector and transformed into DH5 alpha Escherichia coli competent cells (Bethesda Research Laboratories) and designated pSPR38 (Fig. 9). A 2.3 kb EcoRI - HindIII fragment produced the subclone pSPR45; a 5.0 kb BamHI fragment produced the subclone pSPR46; a 3.3 kb PstI fragment produced the subclone pSPR44; a 1.4 kb PstI fragment produced the subclone pSPR42. Additional subclones were produced as deletion products by deleting sequences from the HindIII end of the EcoRI - HindIII DNA fragment in the subclone pSPR45. Once digested with HindIII, Dnase was applied for increasing lengths of time to shorten the fragment. The new end was treated with DNA polymerase and nucleotides were added to blunt the end for ligation into linearized, blunt-ended vectors (pBluescript). By successive treatments, the subclones pSPR51, pSPR54, pSPR57, and pSPR59 were constructed (Fig. 9).
To determine whether the clones were expressing P39, expression assays of the P39 deletion and subclone variants (Fig. 9) were performed with polyclonal anti-P39 serum (anti-pSPR33, previously described), monoclonals A6 and D1 and Western blotted whole-cell lysates. Two monoclonal antibodies to P39 antigen were produced using standard techniques for one of ordinary skill in the art. Escherichia coli cells containing the recombinant pSPR33 were inoculated intraperitoneally into BALB/c laboratory mice. After one month, the mice were boosted with an identical inoculum. One week after the boost, serum samples from the mice were tested by Western blot analysis for anti-P39 antibodies and mice seropositive were again boosted with recombinant E. coli. After three days, spleen were removed. Spleen cells were separated and fused with hybridoma cells SP-20 in HY culture media, 37° C, 8% CO₂. Successful fusions were then cloned by limiting dilution in 96-well microtiter plates. Tissue culture supernatants of positive cell cultures were then tested by Western blot analysis for anti-P39 antibody. Two clones positive for such analysis, designated A6 and D1, were used in subsequent analysis of P39 antigen and the expression of various subclones of pSPR33 as previously described.
To examine various antisera and monoclonal antibodies by Western blot analysis for anti-P39 antibodies, the *E.* coli recombinant with pSPR33 was first lysed by heat in 2-mercaptoethanol and then electrophoresed in a 12.5% SDS-polyacrylamide gel for 6 hr. The gel was then electroblotted with the Towbin system for 3 hr. to transfer the *E. coli* recombinant proteins onto a nitrocellulose membrane. After transfer, the membrane was blocked with TSE-Tween to reduce the nonspecific binding of immunoglobulins. Next the membrane was immersed in the appropriate test serum or monoclonal antibody and incubated at room temperature with rocking for 1 hr. The membrane was then rinsed with water and incubated next in a solution of ¹²⁵I-protein A to label antibodies bound to the antigens on the membrane. After incubation and washing off the excess label, the membrane was dried and placed on Kodak XAR-5 film for autoradiographic detection of the anti-P39 antibodies. Similar assays were conducted for the other subclones.
Plasmids pSPR38 and pSPR46 expressed the same amount of P39 as the primary clone pSPR33. This, along with the fact that plasmid pSPR51 expressed P39 whereas pSPR54 did not, we conclude that the gene for P39 was between the RamHI and HindIII sites (Fig. 9, black bar). The amount of P39 associated with cell lysates of clones pSPR51 and pSPR45 is less than the other clones that were P39 positive. This suggested that sequences to the right of the gene locus were important for full expression. P39 was produced by a clone (pSPR46) that contained the insert in the opposite orientation to that of other P39 producing clones (e.g. pSPR38). Therefore, expression or P39 was not dependent on the Lac promoter (Fig. 9, back arrows). The pst1 fragment that was subcloned from pSPR33 and designated pSPR44 (Fig. 9), did not express detectable amounts of P39. Thus, the P39 gene was assumed to be transcribed from left to right. We presume that the additional sequences correspond to the second of two genes that express similar but distinct antigens and that they collectively augment the amount of antibody that binds the 39 kDa band in the immunoblot assay. Because the plasmid pSPR44 did not express any antigens reactive with polyclonal anti-P39 serum, the expression of the second gene located to the right of the black box (Fig. 9) may depend on the transcription of the first gene.
Example 5. DNA sequencing of the gene encoding P39. The DNA sequence (Fig. 10) was determined for the BamHI-HindIII fragment (Fig. 9, black box) by the strategy summarized in Fig. 11b. Essentially, sequence was obtained using primers designed from DNA sequence determined using the universal M13 primer and the subclones pSPR46, pSPR44, and pSPR45, and the Mung Bead nuclease deletion variants pSPR51, pSPR54, pSPR56, and pSPR57, of plasmid pSPR45. DNA sequence to the right of the HindIII restriction site was determined using primers designed from existing sequence information.
DNA sequence was obtained first by using primers designed for use with the M13 universal primer and available sequence of the cloning vector. The protocol for performing the sequencing reactions was exactly that provided by United States Biochemical (Sequenase - Version 2.0: Step-By-Step Protocols for DNA Sequencing With Sequenase ® Version 2.0 - 5th Edition). Sequencing reactions were run in small plastic centrifuge tubes. Each reaction volume was 10ml and included primer, buffer and DNA to anneal primer to template. Labeling was done by adding Sequenase, ³⁵S-dATP, and additional buffer. Termination of the A, T, G, and C reactions was done by adding a stop solution. Samples were then heated to 70° - 80°C for two minutes and then 2-3 ml of each mix was added to each lane of the gel. All sequencing gels were 6% acrylamide - 7M urea - 1 x TBE and were run for 2hr or 4hr. After running, the gels were fixed in 5% acetic acid - 15% methanol to remove urea. Gels were then dried at 80°C under vacuum then placed on Kodak XAR-5 film. Exposed films were then analyzed for autoradiographic bands to determine the sequence. Terminal sequences of each reaction were used to generate new oligonucleotide primers for use in the next sequencing reactions. Therefore, the entire sequences of each strand of DNA were determined through successive extensions using primers determined by previous reactions. By way of example, synthetic primers of 20 nucleotides from a region of SEQ ID no. 1 can be constructed and utilized to sequence about 300 bases. Other primers can then be constructed from the deduced sequence. Such techniques are standard and would be known to one of ordinary skill in the art.
Analysis of the completed DNA sequence (SEQ. ID No. 1) revealed two open reading frames (Fig. 10a). Gene 1 was in frame 1 and gene 2 was in frame 3. No other significant open reading frames were detected. The DNA sequence has been numbered from the adenine residue of the ATG start codon for the protein encoded by gene 1 because it is assumed that this is the first gene transcribed. This open reading frame (nucleotides 1 to 1020) was confirmed by sequencing the first 15 amino acids of P39 expressed by clone pSPR51. This clone has had gene 2 deleted, and therefore its gene product was not detected during protein sequencing. Gene 1 corresponds to a protein of 339 amino acids with a calculated molecular weight of 36.926 kDa. Because this gene encodes a protein that reacted with all of 10 serum specimens collected from: human Lyme patient but not to 10 normal controls specimens (data not shown), it assumed that this protein is equivalent to P39. Because of the existence of a second gene product with a similar molecular weight that may also bind human serum, it was determined that the P39 antigen as previously described is not one protein but two proteins (39α and 29 β). This is suggested by the expression data shown in Fig. 9, where the P39 signal appears to be enhanced if both genes are present. The open reading frame (nucleotides 1107 to 2132) of gene 2 has been designated p39β. This genes' open reading frame begins 116 nucleotides down stream of p39α and encodes a protein of 341 amino acids (37.506 kDa). A promoter 5' to the start codon in p39α appeared to be present with classic -10 and -35 regions whereas the p39β lacked recognizable promoter sequences. Both genes, however, had putative ribosomal binding sites immediately 5' to the start codons and each was terminated with a TAA codon at positions 1018 and 2130 respectively. The putative promoter and ribosomal binding sites resemble those associated with other genes from *B. burgdorferi* including the opsA-operon and the flagellin gene (Wallich, R., S.E. Moter, M.M. Simon, K. Ebnet, A. Heiberger, and M.D. Kramer, 1990. The *Borrelia burgdorferi* flagellum-associated 41 kilodalton antigen (flagellin); molecular cloning, expression and amplification of the gene. Infect Immun 58:1711-1719). Unlike the genes encoding the flagellin, OspA and OspB, no stem loop structures were detected at the 3' 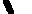 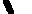 end of either p39α and p39β, suggesting termination may be outside what has been sequenced. Nevertheless, in accordance with the transcription termination regions in may bacteria, including *Borrelia*, this region is AT rich, suggesting that termination is in the vicinity of nucleotide 2170.
Comparing the DNA sequence of p39α and p38β by the Needleman and Munsch global alignment program (Needleman and Munsch, J. Mol. Biol.; 148:443-53 (1970)), indicates that these genes have 62% DNA sequence similarity. No significant sequence similarity was detected between the P39 genes and either the OspA-OspB operon or the flagellin gene. Codon preference and G + C content analysis of the p39 operon indicated that there were no significant differences between it and the other *Borrelia* genes.
Example 6. Determination of p39α and p39β transcript size. Northern blot analysis (Fig. 12) of total RNA from *B. burgdorferi* strains B31 and Sh-2-82 were probed with a PstI-HindIII fragment internal to the p39α and p39β loci (Fig. 11). This probe detected a single 2.35 kb message, and tends to confirm that the P39α and β mRNA is polycistronic and that p39α and p39β constitute an operon (p39). This conclusion is supported by the DNA sequence data described above which shows that p39β does not appear to have a recognizable promoter. Furthermore, this explains why clones that carry an intact p39β but lack the promoter for p39α (eg. pSPR44), do not express antigens reactive with polyclonal anti-P39 serum (anti-pSPR33) (Fig. 9). As a control for specificity, total RNA from E. coli was shown not to hybridize to the PstI-HindIII *Borrelia* fragment (Fig. 12). The amino acid composition of P39α and P39β are similar (SEQ. ID. No. 2 and 3, Table 4), although distinct from the amino acid composition of OspA and OspB. P39 and P39β contained comparatively much larger amounts of isoleucine, proline, arginine, phenylalanine, tyrosine, and methionine. Furthermore, lysine and threonine, which are present in large amounts in OspA and OspB, constitute a much smaller proportion of P39α and P39β. Between P39α and P39β, the major difference was the 3 cysteine residues in the later protein and 4 histidine residues in the former protein (Table 4).

**Table 4**

| Amino acid composition of proteins encoded by the P39 operon | | |
|---|---|---|
| | P39α(%) | P39β(%) |
| Alanine | 25 (7.4) | 22 (6.5) |
| Cysteine | 1 (0.3) | 3 (0.9) |
| Aspartic acid | 20 (5.9) | 21 (6.2) |
| Glutamic acid | 26 (7.7) | 21 (6.2) |
| Phenylalanine | 16 (4.7) | 16 (4.7) |
| Glycine | 33 (9.7) | 32 (9.4) |
| Histidine | 4 (1.2) | 1 (0.3) |
| Isoleucine | 37 (10.9) | 43 (7.6) |
| Lysine | 30 (8.8) | 26 (7.6) |
| Leucine | 32 (9.4) | 26 (7.6) |
| Methionine | 5 (1.5) | 6 (1.8) |
| Asparagine | 17 (5.0) | 21 (6.2) |
| Proline | 8 (2.4) | 7 (2.1) |
| Glutamine | 4 (1.2) | 7 (2.1) |
| Arginine | 7 (2.1) | 9 (2.6) |
| Serine | 29 (8.6) | 30 (8.8) |
| Threonine | 12 (3.5) | 5 (1.5) |
| Valine | 18 (5.3) | 25 (7.3) |
| Tryptophan | 1 (0.3) | 2 (0.6) |
| Tyrosine | 14 (4.1) | 18 (5.3) |
| | 339 | 341 |

P39β, like OspA and OspB, has a classic signal peptide including the putative cleavage site defined by the tetrapeptide Leu-X-X-Cys (Fig. 13), where X usually represents any neutral amino acid. For P39β, the leu residue is at position 12 and the cysteine at position 15 (SEQ. ID. No. 1). Although P39α also has a hydrophobic N-terminus (Fig. 14) and a cysteine at a similar position (position 18), this protein does not have the tetrapeptide, suggesting that its putative signal sequence is processed in a different manner to that of the corresponding region in P39β. Because P39α and P39β have a cysteine at close to the same position as the cysteine in OspA and OspB, and it has been predicted that the latter two proteins are acylated at the site, P39α and P39β may also be lipoproteins due to acylation of their N-terminal cysteine residue.
Comparing the amino acid sequence of P39α and P39β revealed 52% sequence identity. This is similar to the reported 53% similarity between OspA and OspB. Surprisingly and in contrast to that found for OspA and OspB, the p39 operon proteins have very similar hydropathy plots (Fig. 14). This, along with the high degree of sequence similarity, indicates that the two proteins share a considerable number of the same epitopes having immunogenic properties. Antiserum raised to OspA will react to OspB, indicating proteins like P39α and P39β with significant identity at the amino acid level will share cross-reactive epitopes.
The genetic element encoding the immunodominate antigen P39 was identified and sequenced. This element was shown to be two genes that constituting an operon encoding two similar sized proteins, P39α and P39β, that have considerably amino acid sequence similarity. This is the first report of an operon encoding putative membrane proteins that has a chromosomal origin in *B. burgdorferi*. It is assumed that both the α and β forms contribute to the signal when antibody from infected animals binds the P39 band in Western blots (Simpson, W.J., W. Burgdorfer, M.E. Schrumpf, R.H. Karstens, and T.G. Schwan, 1991. Antibody to a 39 kDa *Borrelia burgdorferi* antigen (P39) as a marker for infection in experimentally and naturally inoculated animals. J Clin Microbio 29:236-243. Simpson, W.J., M.E. Schrumpf, and T.G. Schwan, 1990. Reactivity of human Lyme borreliosis sera with a 39-kilodalton antigen specific to *Borrelia burgdorferi*. *J Clin Microbiol 28:1329-1337*). This raises the question of whether all Lyme serum reacts equally well to both α and β forms or whether some serum reacts to one and not the other.
The function of P39α and P39β is not known, but several characteristics, deduced from the predicted amino acid sequence suggests several possibilities. These proteins exhibit alternating hydrophobic and hydrophilic regions, characteristic of an amphophilic or transmembrane protein. In accordance with a membrane location, immune electron microscopy analyses of *B. burgdorferi* with monoclonal antibody A6 indicates that the P39 antigen is in or associated with the membranes (unpublished data).
P39β resembles OspA and OspB in that it has typical signal sequence and cleavage site at the first cysteine residue. Like OspA and OspB, P39β is probably membrane associated and may be acrylated at the N-terminal cysteine. P39α, however, is different with regard to its signal sequence which may not be cleaved because it lacks the type 1 recognition site. If so, P39α may be secreted and therefore the antigen that stimulates the immune response during an infection. This notion would help to explain the earlier observation that anti-P39 antibodies appear to more readily associated with the infected state, because a secreted form could accumulate more rapidly during the early stages of an infection than that associated with cells. (Simpson, W.J., W. Burgdorfer, M.E. Schrumpf, R.H. Karstens, and T.G. Schwan, 1991. Antibody to a 39 kDa *Borrelia burgdorferi* antigen (P39) as a marker for infection in experimentally and naturally inoculated animals. J Clin Microbiol 29:236-243.)
Example 6: Amplification of gene 1 (P39α) and gene 2 (P39β)
To determine the immunoreactiveness both P39α and P39β, gene 1 and gene 2 will be cloned separately and the expression products examined for their reactivity with Lyme immune sera. Standard methodologies for cloning and expressing each gene can be employed; however, it is preferred to amplify each gene separately using the polymerase chain reactive (PCR) and the primer sequences identified in SEQ. ID Nos. 4-7.
The synthetic oligonucleotide DNA primers described were constructed with an Applied Biosystems Inc. DNA Synthesizer Model 380-B, following the instructions provided by the manufacturer. In this procedure, short chains of nucleotides of a specific order are produced in a concentrated ammonium hydroxide solution. This material is then centrifuged under vacuum to remove the ammonium hydroxide. The dry DNA pellet is then resuspended in TE buffer and the DNA concentration is determined by spectrophotometric absorbance at 260 mm. Concentrations of the DNA primers are then standardized for PCR according to the protocol provided by Perkin-Elmer-Cetus.
To amplify *B. burgdorferi* DNA by PCR using the primers described, the protocol involves mixing the *B. burgdorferi* DNA with either primers 1 and 2 for gene 1 (sequences 4 and 5), primers 1 and 2 for gene 2 (sequences 6 and 7), and sequences 4 and 7 to amplify both genes 1 and 2 together. Also added to the PCR mix is the DNA *Taq* polymerase, buffer, and the mixture of the four nucleotides (dNTPs). This reaction mixture is then subjected to repetitive cycles of three different temperatures to cause denaturing the DNA, annealing of the primers to the template DNA, and extension (polymerization) to produce a new strand of DNA. After 30 cycles using the thermal cycler, the PCR amplification products are examined by running 10 µl of each sample in an electrophoresis agarose gel.
In order that the amplified products can be inserted into known vectors by standard techniques known to a skilled artisan, at the 5' end of each primer, nucleotides will be added that encode for the recognition site for the restriction endonuclease EcoRI (G/AATTC).
The amplified DNA products will be comprised of each gene with the addition of an EcoRI site at each end, which will allow us to insert this sequence into any one of many available cloning and expression vectors which have only one EcoRI site available, such as pUC, pBluescript, pBR322, etc. The vectors are inserted into host cells to obtain expression of the DNA products. Such techniques are well known to one of ordinary skill in the art.
Next, recombinants having the appropriate sized inserted DNA (1017 bases for gene 1; 1023 bp for gene 2) will be examined by Southern blot analysis to identify the cloned fragments. DNA from recombinants with the presumptive gene 1 or gene 2 will be separated in agarose gels, transferred to nitrocellulose membranes, and probed with the purified EcoRI fragment from pSPR33. Such procedures are standard techniques well known to anyone skilled in the art. After confirming that the amplified cloned fragments are homologous with the pSPR33 insert, the various clones are tested for expression of P39 antigens using standard Western immunoblotting techniques. Rabbit anti-pSPR33 antiserum, anti-P39 monoclonal antibodies (as previously described), and convalescent serum from human Lyme patients will each be reacted with whole-cell lysates of the various clones to identify and obtain expression products of each gene. The synthetic peptides can be mapped to identify specific immunoreactive epitopes, used in bioassays to detect Lyme borreliosis disease or used in vaccines for mammals against Lyme borreliosis disease.

## Claims

1. A protein available from Borrelia burgdorferi, having a molecular weight of about 39 kilodalton as determined by SDS-PAGE, or a portion thereof, having specific reactivity with mammalian Lyme borreliosis serum but not with sera from patients suffering other spirochete-based diseases, wherein the protein is expressed from a DNA fragment comprising the nucleotide sequence shown in any of SEQ ID Nos. 1, 2 and 3.

2. A protein according to claim 1, having the amino-acid sequence shown in SEQ ID No. 2.

3. A protein according to claim 2, having the amino-acid sequence shown in SEQ ID No. 3.

4. A protein according to any preceding claim, wherein the mammal is a human.

5. A DNA fragment encoding a protein as defined in any of claims 1 to 4.

6. A DNA fragment comprising the nucleotide sequence shown in SEQ ID No. 1 or a mutant thereof.

7. A DNA fragment comprising the nucleotide sequence shown in SEQ ID No. 2 or a mutant thereof.

8. A DNA fragment comprising the nucleotide sequence shown in SEQ ID No. 3 or a mutant thereof.

9. A recombinant DNA molecule, e.g. pSPR33, comprising a DNA fragment according to any of claims 5 to 8 and a vector, e.g. pBluescript SK.

10. A host cell stably transformed with a recombinant DNA molecule according to claim 9 and capable of expression of protein encoded in the DNA fragment.

11. A host cell according to claim 10, which is Escherichia coli.

12. A method of producing a Borrelia burgdorferi 39 kD protein, comprising culturing host cells according to claim 10 or claim 11, and isolating the protein.

13. A complex comprising a protein as defined in any of claims 1 to 4 bound to a solid support.

14. An antibody specific for a protein according to any of claims 1 to 4.

15. An antibody according to claim 14, which is monoclonal.

16. An antibody according to claim 14, which is polyclonal.

17. A complex comprising an antibody according to any of claims 14 to 16 bound to a solid support.

18. A vaccine for mammals against Lyme borreliosis disease, comprising a protein according to any of claims 1 to 4, and a pharmaceutically-acceptable carrier.

19. A vaccine according to claim 18, which further comprises an adjuvant.

20. A bioassay for the diagnosis of Lyme borreliosis disease, comprising the steps of:
i) coating a surface with a protein according to any of claims 1 to 4;
ii) contacting the coated surface with serum from a mammal suspected of having Lyme disease; and
iii) detecting the presence or absence of a complex formed between the protein and antibodies specific therefor present in said serum.

21. A bioassay for the diagnosis of Lyme borreliosis disease, comprising the steps of:
i) coating a surface with an antibody according to any of claims 14 to 16;
ii) contacting the coated surface with serum from a mammal suspected of having Lyme disease; and
iii) detecting the presence or absence of a complex formed between the antibodies and proteins present in the serum.

22. The method according to claim 20 or claim 21, wherein the surface is a gel, slide, membrane or microtitration plate.

23. A diagnostic kit comprising a protein according to any of claims 1 to 4 and ancillary reagents suitable for use in detecting the presence of antibodies to the protein in a mammalian blood or other tissue sample.

24. A method of screening a drug for anti-Lyme borreliosis disease activity, comprising contacting the drug with cells contacted with Borrelia burgdorferi under conditions such that inhibition of expression of the protein according to any of claims 1 to 4 can be effected.

## Patentansprüche

1. Aus Borrelia burgdorferi erhältliches Protein mit einem Molekulargewicht von, durch SDS-PAGE bestimmt, etwa 39 Kilodalton oder einem Teil hiervon, und mit spezifischer Reaktivität mit Säugetier-Lyme-Borreliose-Serum, jedoch nicht mit Seren von Patienten, die an anderen durch Spirochaeten verursachten Krankheiten leiden, wobei das Protein aus einem DNA-Fragment exprimiert ist, das die Nukleotidsequenz aufweist, wie sie unter einer der Sequenz-Identifikationsnummern 1, 2 und 3 dargestellt ist.

2. Protein nach Anspruch 1, mit der unter der Sequenz-Identifikationsnummer 2 dargestellten Aminosäuresequenz.

3. Protein nach Anspruch 2, mit der unter der Sequenz-Identifikationsnummer 3 dargestellten Aminosäuresequenz.

4. Protein nach einem der vorhergehenden Ansprüche, wobei der Säuger ein Mensch ist.

5. DNA-Fragment, das ein Protein nach einem der Ansprüche 1 bis 4 kodiert.

6. DNA-Fragment, das die Nukleotidsequenz, wie sie unter Sequenz-Identifikationsnummer 1 dargestellt ist, oder einen Mutanten davon enthält.

7. DNA-Fragment, das die Nukleotidsequenz, wie sie unter Sequenz-Identifikationsnummer 2 dargestellt ist, oder einen Mutanten davon enthält.

8. DNA-Fragment, welches die Nukleotidsequenz, wie sie unter Sequenz-Identifikationsnummer 3 dargestellt ist, oder einen Mutanten davon enthält.

9. Rekombinantes DNA-Molekül, zum Beispiel pSPR33, das ein DNA-Fragment nach einem der Ansprüche 5 bis 8 und einen Vektor, zum Beispiel pBluescript SK, enthält.

10. Wirtszelle, die mit einem rekombinanten DNA-Molekül nach Anspruch 9 stabil transformiert und in der Lage ist, ein in dem DNA-Fragment kodiertes Protein zu exprimieren.

11. Wirtszelle nach Anspruch 10, bei der es sich um Escherichia coli handelt.

12. Verfahren zum Herstellen eines Borrelia burgdorferi-39-kD-Protein, bei welchem Wirtszellen nach Anspruch 10 oder 11 gezüchtet und das Protein isoliert wird.

13. Komplex, der ein an einen festen Träger gebundenes Protein nach einem der Ansprüche 1 bis 4 aufweist.

14. Für ein Protein nach einem der Ansprüche 1 bis 4 spezifischer Antikörper.

15. Antikörper nach Anspruch 14, der monoklonal ist.

16. Antikörper nach Anspruch 14, der polyklonal ist.

17. Komplex mit einem Antikörper nach einem der Ansprüche 14 bis 16, der an einen festen Träger gebunden ist.

18. Impfstoff für Säugetiere gegen Lyme-Borreliose-Krankheit, der ein Protein nach einem der Ansprüche 1 bis 4 und einen pharmazeutisch annehmbaren Träger enthält.

19. Impfstoff nach Anspruch 18, der außerdem ein Adjuvans enthält.

20. Biologischer Test zur Diagnose von Lyme-Borreliose-Krankheit, bestehend aus folgenden Schritten:
i) Beschichten einer Oberfläche mit einem Protein nach einem der Ansprüche 1 bis 4,
ii) In-Berührung-Bringen der beschichteten Oberfläche mit Serum eines Säugers, der mutmaßlich Lyme-Krankheit hat, und
iii) Feststellen der Anwesenheit oder Abwesenheit eines zwischen dem Protein und dafür spezifischen, in dem Serum vorhandenen Antikörpern gebildeten Komplexes.

21. Biologischer Test zur Diagnose von Lyme-Borreliose-Krankheit, mit folgenden Schritten:
i) Beschichten einer Oberfläche mit einem Antikörper nach einem der Ansprüche 14 bis 16,
ii) In-Berührung-bringen der beschichteten Oberfläche mit Serum eines Säugers, der mutmaßlich Lyme-Krankheit hat, und
iii) Feststellen der Anwesenheit oder Abwesenheit eines zwischen den Antikörpern und in dem Serum vorhandenen Proteinen gebildeten Komplexes.

22. Verfahren nach Anspruch 20 oder 21, wobei die Oberfläche ein Gel, ein Objektträger, eine Membran oder ein Mikrotiterplättchen ist.

23. Diagnoseausrüstung, die ein Protein nach einem der Ansprüche 1 bis 4 und Hilfsreagentien enthält, die zur Verwendung beim Nachweis der Anwesenheit von Antikörpern zu dem Protein in Säugetierblut oder einer anderen Gewebeprobe geeignet sind.

24. Verfahren zum Prüfen eines Arzneimittels auf Anti-Lyme-Borreliose-Aktivität, wobei das Arzneimittel mit Zellen, die mit Borrelia burgdorferi Berührung hatten, unter solchen Bedingungen in Berührung gebracht wird, daß eine Hemmung der Expression des Proteins nach einem der Ansprüche 1 bis 4 bewirkt werden kann.

## Revendications

1. Protéine obtenable à partir de Borrelia burgdorferi, ayant un poids moléculaire d'environ 39 kilodaltons tel que déterminé par SDS-PAGE, ou une partie de celle-ci, ayant une réactivité spécifique avec du sérum de mammifère atteint de la borréliose de Lyme mais non avec des sérums provenant de patients souffrant d'autres maladies provoquées par des spirochètes, dans laquelle la protéine est exprimée à partir d'un fragment d'ADN comprenant la séquence nucléotidique représentée dans l'une des SEQ ID Nos. 1, 2 et 3.

2. Protéine suivant la revendication 1, comportant la séquence d'acides aminés représentée dans la SEQ ID No. 2.

3. Protéine suivant la revendication 2, comportant la séquence d'acides aminés représentée dans la SEQ ID No. 3.

4. Protéine suivant l'une quelconque des revendications précédentes, dans laquelle le mammifère est un être humain.

5. Fragment d'ADN codant pour une protéine telle que définie dans l'une quelconque des revendications 1 à 4.

6. Fragment d'ADN comprenant la séquence nucléotidique représentée dans la SEQ ID No. 1 ou un mutant de celui-ci.

7. Fragment d'ADN comprenant la séquence nucléotidique représentée dans la SEQ ID No.2 ou un mutant de celui-ci.

8. Fragment d'ADN comprenant la séquence nucléotidique représentée dans la SEQ ID No.3 ou un mutant de celui-ci.

9. Molécule d'ADN recombinant, par exemple pSPR33, comprenant un fragment d'ADN suivant l'une quelconque des revendications 5 à 8 et un vecteur, par exemple pBluescript SK.

10. Cellule hôte transformée de façon stable avec une molécule d'ADN recombinant suivant la revendication 9 et pouvant exprimer une protéine codée dans le fragment d'ADN.

11. Cellule hôte suivant la revendication 10, qui est de l'Escherichia coli.

12. Procédé de production d'une protéine de 39 kD de Borrelia burgdorferi, comprenant la culture de cellules hôtes suivant la revendication 10 ou la revendication 11 et l'isolement de la protéine.

13. Complexe comprenant une protéine telle que définie dans l'une quelconque des revendications 1 à 4 liée à un support solide.

14. Anticorps spécifique pour une protéine suivant l'une quelconque des revendications 1 à 4.

15. Anticorps suivant la revendication 14, qui est monoclonal.

16. Anticorps suivant la revendication 14, qui est polyclonal.

17. Complexe comprenant un anticorps suivant l'une quelconque des revendications 14 à 16 lié à un support solide.

18. Vaccin pour mammifères contre la maladie borréliose de Lyme, comprenant une protéine suivant l'une quelconque des revendications 1 à 4 et un support pharmaceutiquement acceptable.

19. Vaccin suivant la revendication 18, qui comprend de plus un adjuvant.

20. Bioessai pour le diagnostic de la maladie borréliose de Lyme, comprenant les étapes suivantes :
i) le recouvrement d'une surface avec une protéine suivant l'une quelconque des revendications 1 à 4 ;
ii) la mise en contact de la surface recouverte avec du sérum provenant d'un mammifère suspecté d'avoir la maladie de Lyme ; et
iii) la détection de la présence ou de l'absence d'un complexe formé entre la protéine et les anticorps spécifiques de celle-ci présents dans le sérum.

21. Bioessai pour le diagnostic de la maladie borréliose de Lyme, comprenant les étapes suivantes :
i) le recouvrement d'une surface avec un anticorps suivant l'une quelconque des revendications 14 à 16 ;
ii) la mise en contact de la surface recouverte avec du sérum provenant d'un mammifère suspecté d'avoir la maladie de Lyme ; et
iii) la détection de la présence ou de l'absence d'un complexe formé entre les anticorps et les protéines présents dans le sérum.

22. Procédé suivant l'une ou l'autre des revendications 20 et 21, dans lequel la surface est un gel, une lamelle, une membrane ou une plaque de microtitrage.

23. Trousse de diagnostic comprenant une protéine suivant l'une quelconque des revendications 1 à 4 et des réactifs auxiliaires convenant pour être utilisés dans la détection de la présence d'anticorps à la protéine dans un échantillon de sang ou tout autre échantillon de tissu de mammifère.

24. Procédé pour sélectionner un médicament pour son activité anti-maladie ou borréliose de Lyme, comprenant la mise en contact du médicament avec des cellules ayant été mises en contact avec Borrelia burgdorferi sous des conditions telles qu'une inhibition de l'expression de la protéine suivant l'une quelconque des revendications 1 à 4 puisse être effectuée.
